# EUROPEAN PATENT SPECIFICATION

(11) **EP 4 307 899 B1**
(45) Date of publication and mention of the grant of the patent: **27.05.2026**
(21) Application number: 22771948.1
(22) Date of filing: 10.03.2022
(51) Int. Cl.: A01N 59/20, A01N 59/16, A61L 27/30, B82Y 30/00, C02F 1/50, C04B 41/51

(54) **BIOFILM-RESISTANT ARTICLES COATED WITH METAL NANOPARTICLE AGGLOMERATES**
BIOFILMRESISTENTE ARTIKEL MIT METALLNANOPARTIKELAGGLOMERATEN
ARTICLES RÉSISTANTS AU BIOFILM REVÊTUS D'AGGLOMÉRATS DE NANOPARTICULES MÉTALLIQUES

(30) Priority: 15.03.2021 US 202163160998 P
(43) Date of publication of application: 24.01.2024
(73) Proprietor: Kuprion Inc., San Jose, CA 95134 (US)
(72) Inventor: ZINN, Alfred A., Palo Alto, CA 94303 (US); BRODY, Rachel, Mountain View, CA 94040 (US)
(74) Representative: Novagraaf Group
(86) International application number: PCT/US2022/019692
(87) International publication number: WO 2022/197517

(56) References cited:
- WO-A1-2010/104806
- US-A1- 2007 207 335
- US-A1- 2008 063 693
- US-A1- 2013 091 611
- US-A1- 2016 201 183
- MOHAMED ASEM A ET AL: "Eco-friendly Mycogenic Synthesis of ZnO and CuO Nanoparticles for In Vitro Antibacterial, Antibiofilm, and Antifungal Applications", BIOLOGICAL TRACE ELEMENT RESEARCH, HUMANA PRESS, CLIFTON, NJ, US, vol. 199, no. 7, 8 September 2020 (2020-09-08), pages 2788 - 2799, XP037515304, ISSN: 0163-4984, [retrieved on 20200908], DOI: 10.1007/S12011-020-02369-4

## Description

### BACKGROUND

Biofilms are groupings of bacteria that have aggregated on a surface and begun to synthesize extracellular polymeric substances that affix the bacteria firmly to the surface and to each other. The bacteria within a given biofilm may represent a substantially homogeneous bacterial population or a diverse range of different types of bacteria. The latter situation is very common. Bacteria within a biofilm are phenotypically different than are those of freely suspended planktonic bacteria, as the bacteria within a biofilm may perform rudimentary molecular signaling amongst each other through plasmid transfer. Gene regulation and transcription are also altered relative to their planktonic counterparts. As such, biofilms represent a matrix of single-celled organisms, potentially acting in cooperation (symbiosis) with each other and exchanging information, rather than a true multi-celled organism.

Overall, the structure of biofilms may vary widely in composition and complexity. The extracellular polymeric substance of biofilms comprises predominantly polysaccharides, typically neutral or anionic polysaccharides in the case of gram-negative bacteria and cationic polysaccharides in the case of gram-positive bacteria. Anionic polysaccharides may arise from attachment of uronic acids or ketal-linked pyruvates to the polysaccharide backbone, which may allow crosslinking to occur through complexation with divalent metal cations such as Ca²⁺ and Mg²⁺. Approximately 50-60% of the total organic carbon in biofilms arises from extracellular polymeric substances. The extracellular polymeric substances within biofilms provide a barrier between the bacteria and their external environment, thereby making the bacteria especially difficult to remediate using conventional agents, such as antibiotic substances. Depending on their location, additional components within the matrix of a biofilm may include, for example, mineral crystals, blood components, and the like. Association with other biological substances such as proteins, DNA, lipids and/or humic substances may also occur. Significant amounts of water may also be present.

Any type of solid-liquid interface may experience growth of a biofilm. Especially susceptible surfaces may include those that are rougher due to their lower shear forces and higher surface area, and hydrophobic, non-polar surfaces such as polymers. Glass and metal surfaces are somewhat more resistant to biofilm formation, but they too usually become susceptible to biofilm formation over time. Certain liquids, such as biological fluids (e.g., saliva, blood, plasma, interstitial fluid, urine, tears, intravascular fluid, respiratory secretions, and the like) and most types of aqueous media contain trace or non-trace amounts of polymers that may deposit upon a surface and provide a conditioning film that may stimulate biofilm growth. The type of conditioning film in contact with a surface may dictate the rate and extent of biofilm formation. Additional factors impacting biofilm growth may include, for example, flow velocity in proximity to a surface, pH, nutrient levels, ionic strength, and temperature.

Greater than an estimated 80% of infections in humans involve biofilms, and they may not be effectively cleared by the immune system. Medical implants, such as catheters, central lines, IVs, stents, joint replacements, valve replacements, pacemakers, and other types of medical devices represent surfaces that may be especially susceptible toward biofilm growth. Bandages, sensors, and other medical equipment placed upon the skin are also susceptible to biofilm formation. In addition, biofilms may even occur natively within the body, with plaque formation upon teeth being a familiar example.

If not effectively treated, biofilms within a human may progress to various types of infections such as endocarditis, pneumonia, blood stream infections, wound infections, laryngitis, and the like. Embolisms resulting from biofilm detachment and endotoxin formation from gram-negative bacteria may also be problematic. Infections may be delayed as much as a year following introduction of an implant and may be difficult to treat at that point. Antibiotics administered at maintenance doses may keep biofilm formation somewhat under control, but the doses may be problematic for the long-term health of a patient. Antibiotic resistance may also become more prevalent over time. Beyond antibiotics, the only effective solution for ridding a patient of biofilms is often mechanically removing the biofilm, such as through debriding/sloughing a wound or other surface or removing and replacing a medical implant, both of which may be costly and themselves present an undesirable health insult. Hydrophilic or superhydrophilic coatings applied to an implant before introduction to a patient may temper biofilm formation at least to some degree, but some types of surfaces may not be effectively treated with these types of coatings and doing so may increase production costs.

In addition to the medical space, biofilms may be present in a number of environmental settings where they may result in effects ranging from mere nuisance to causing significant damage. Non-limiting examples of locations where environmental biofilms may occur include, for example, boat hulls, submerged piers, pipelines and drains, oil production facilities, deep sea oil rigs, ornamental ponds and fountains, aquariums, swimming pools, food storage facilities, food processing facilities, slaughterhouses, water heaters, showers, bathtubs, toilets, kitchen gadgets such as sponges, children's toys, and the like. Like biofilms occurring internally within a human, environmental biofilms, once formed, may be most effectively removed through mechanical processes, often at significant expense.

Mohamed et al (Biological Trace Element Research (2021) 199:2788-2799) discloses a mycogenic synthesis of ZnO and CuO nanoparticles for *in vitro* antibacterial, antibiofilm, and antifungal applications.

US 2016/0201183 A1 discloses an article having antiseptic properties. The article includes an exposed surface coating containing a plurality of copper islands, and a substructure underlying the exposed surface coating. The substructure contains a material differing from copper.

### SUMMARY

In one aspect, the present invention provides a biofilm-resistant article according to claim 1. In another aspect, the present invention provides a method of using the biofilm resistant article according to claim 11. Certain more specific aspects of the invention are set out in the dependent claims.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, as will occur to one having ordinary skill in the art and the benefit of this disclosure.
FIGS. 1 and 2 show diagrams of presumed structures of metal nanoparticles having a surfactant coating thereon.
FIG. 3 shows an illustrative SEM image of substantially individual copper nanoparticles.
FIG. 4 shows an illustrative SEM image of an agglomerate of copper nanoparticles.
FIG. 5 shows an illustrative SEM image of a copper nanoparticle network obtained after fusion of a plurality of copper nanoparticles to each other.
FIGS. 6A and 6B show illustrative SEM images of agglomerates of copper nanoparticles disposed upon fibers.
FIG. 7 shows an illustrative photographic image of a fabric having agglomerates of copper nanoparticles adhered thereto, as fabricated (left side of image) and after extended use (right side of image).

### DETAILED DESCRIPTION

The present disclosure relates to coatings for inhibiting biofilm formation and proliferation, and more particularly, articles adapted for at least periodic fluid contact having an at least partial coating formed from metal nanoparticles in an amount effective to inhibit biofilm formation and proliferation.

As discussed above, biofilm formation may be problematic in a number of circumstances, both internally within a human and in various external environments. Some types of surfaces are more susceptible toward biofilm formation than are others. However, rendering a surface resistant toward biofilm formation may be problematic in various respects. Some coatings may not adhere adequately over the long term to an article susceptible to biofilm formation when the article's surface is in contact with a fluid, and certain types of coatings may be expensive or difficult to apply.

Accordingly, the present disclosure provides biofilm-resistant articles having a coating comprising metal nanoparticles upon a surface thereof. The coating is applied to at least a portion of the surface of the article and may at least partially inhibit the formation or proliferation of a biofilm thereon. The coating may be applied to the surface of the article during or after manufacturing thereof, including when the article is deployed in its typical use environment.

In particular, the present disclosure provides articles having an at least partial coating of metal nanoparticle agglomerates upon a surface thereof, wherein the metal nanoparticle agglomerates are firmly adhered to the surface of the article and prevent or severely limit the formation and growth of biofilms thereon. Provided there is a good distribution of metal nanoparticle agglomerates upon the surface, even partial coatings may be effective for mitigating biofilm proliferation and growth according to the disclosure herein. Once bacteria contact the metal nanoparticle agglomerates, they may be killed or become inactivated, often through cell wall destruction, such that they are no longer capable of forming a biofilm. Thus, the coating produces the opposite effect of a biofilm disposed upon a surface; instead of being protected by various biofilm matrix components, bacteria die or become inactivated at locations that might otherwise be susceptible to biofilm formation. Surprisingly, killing or inactivation of the bacteria within a biofilm may take place in spite of the biofilm matrix components that may otherwise protect the bacteria from their surrounding environment. Surfactants associated with the metal nanoparticles and metal ions released therefrom may aid in penetrating a biofilm and promoting diffusion therein.

Many types of metal nanoparticles may be used effectively for preventing or mitigating biofilm formation in the disclosure herein. While copper and other bulk metal surfaces are known for their biocidal activity, it may be difficult in practice to introduce metals to the surface of various types of articles susceptible to biofilm formation due to the very high melting point of most metals. In addition, bulk metal surfaces may not have biocidal activity sufficient to preclude biofilm formation. Metal nanoparticles and their agglomerates, such as copper and/or silver nanoparticles, including partially oxidized forms thereof, may be readily introduced to surfaces and especially effective for preventing or limiting growth of biofilms upon the article. Copper nanoparticles are especially advantageous compared to coating an article with molten copper, which forms at the melting point of copper (1083°C), a temperature which is completely incompatible with many materials. Coatings formed through chemical or electrochemical reduction of a copper salt may similarly be difficult to form on various articles. Micron-size metal particles or flakes likewise may be difficult to introduce to an article with sufficient adherence to afford robust performance for inhibiting biofilm formation and proliferation. In addition, the biocidal activity of micron-size metal particles or flakes may not be much different than the biocidal activity of a bulk metal surface, which may afford rather slow bacterial inactivation or killing. The slow rate of inactivation or killing of bacteria by bulk metal surfaces may still leave open the possibility of biofilm formation and proliferation upon these types of surfaces.

Metal nanoparticles, particularly metal nanoparticle agglomerates, represent an effective vehicle for introducing metals onto various articles in a highly active form effective for inhibiting biofilm formation and proliferation. Copper nanoparticles and/or silver nanoparticles may be particularly effective in this respect, given the known biocidal activity of these metals. Cu₂O formed through at least partial oxidation of copper nanoparticles may be particularly effective at inhibiting biofilm formation, especially in combination with metallic copper. Metal nanoparticles and their agglomerates may afford robust adherence to a surface following application thereto. Additional adherence may be afforded through use of an adhesive, which may be chosen from among biologically compatible adhesives for internal (*in vivo*) or similar medical applications. Copper nanoparticles and other types of metal nanoparticles may further be used in combination with one another, which may afford complementary biocidal activity against the same or different types of bacteria comprising a biofilm. Silver nanoparticles may be utilized in combination with copper nanoparticles in some instances, for example, within metal nanoparticle agglomerates. Zinc, nickel, titanium, cobalt, and other bioactive metals may be utilized in further combination with either of these metals as well, including their respective oxides and/or oxides of copper and/or silver, as well as with other additive substances that may convey biocidal activity against biofilm formation. Cu₂O formed from copper nanoparticles, optionally in combination with metallic copper that has not undergone oxidation, may be particularly effective for conveying biocidal activity against biofilm formation.

Metal nanoparticle agglomerates comprise a plurality of fused, partially fused, and/or unfused metal nanoparticles that are associated with one another upon the surface. As discussed further below, agglomerated forms of metal nanoparticles may convey a time-release profile of individual metal nanoparticles, smaller nanoparticle agglomerates, metal ions, and/or metal ion clusters upon the surface of an article subject to biofilm formation, which may afford a highly active metal form in proximity to a forming biofilm, while also limiting toxicity toward mammalian cells. In particular examples, at least a portion of the metal nanoparticles within the metal nanoparticle agglomerates may be unfused with one another to facilitate release of individual metal nanoparticles. Oxidized forms of metal nanoparticles within metal nanoparticle agglomerates may be present upon a surface subject to biofilm formation as well. It is to be appreciated that use of the term "metal nanoparticles" herein may, depending on context, refer to individual metal nanoparticles, agglomerates of metal nanoparticles, or combinations of individual metal nanoparticles and metal nanoparticle agglomerates. Thus, any embodiment disclosed herein may employ metal nanoparticle agglomerates, even if not expressly stated in the description below.

Metal nanoparticles are uniquely situated to promote biocidal activity since they are readily capable of inactivating a range of bacteria and precluding biofilm formation, are low toxicity to humans, especially in small amounts, and may be readily processed into spray formulations, dip coating formulations, and other types of liquid compositions that may facilitate dispensation onto the surface of an article. Metal nanoparticles, such as copper nanoparticles and/or silver nanoparticles, as described further herein, can be readily produced as individual metal nanoparticles and/or agglomerated forms thereof that have a size range compatible with their introduction upon the surface of various articles subject to biofilm formation. The small size of the metal nanoparticles and their agglomerates allows ready distribution upon the surface of the article, optionally in a non-uniform, localized or gradient fashion, to provide protection against biofilm formation and proliferation. Furthermore, due to their high surface energy, metal nanoparticles and their agglomerates may become adhered to the surface of an article following deposition thereon, thereby providing a robust structure that is capable of repeated handling during operational use and long-lived protection against biofilm formation and proliferation. Adherence of the metal nanoparticles and agglomerates thereof may involve chemical bond formation once the metal nanoparticles have attained a high surface energy state, as discussed further herein. As discussed subsequently, an adhesive within the coating upon the surface of an article may alternately promote adherence of metal nanoparticle agglomerates thereto (or supplement the adherence resulting from the high surface energy of the metal nanoparticles within metal nanoparticle agglomerates).

Adhesives may be incorporated upon the surface of an article prior to, after, or while applying metal nanoparticle agglomerates to the surface of an article to promote improved adherence of the metal nanoparticle agglomerates. The adhesive, which may be permanently tacky, may be applied concurrently with the metal nanoparticles or separately, including within a different coating formulation than that used to deposit the metal nanoparticle agglomerates. Application of an adhesive to an article or a portion thereof prior to deposition of metal nanoparticle agglomerates thereon may afford initial sequestration of the metal nanoparticle agglomerates during loading, before more robust adherence is realized through further processing of the surface and the metal nanoparticles occurs, as discussed further below. As another advantage, in addition to binding, trapping, and dispersing the metal nanoparticle agglomerates, the adhesive may further promote prolonged release of active and highly mobile metal species from the metal nanoparticle agglomerates following their adherence to a surface. The active and highly mobile metal species may include individual metal nanoparticles, smaller agglomerates of metal nanoparticles, metal ions, and/or metal ion clusters. By releasing active metal species gradually, the risk of mammalian toxicity may be limited still further while continuing to provide sufficient free metal nanoparticles for inhibiting biofilm formation and proliferation.

Advantageously, metal nanoparticles or agglomerates thereof can be applied to an article through various techniques such as spraying, dip coating, painting, roller coating, printing, stenciling, or the like, thereby affording ready control of the extent of metal loading and the locations where a coating with metal nanoparticle agglomerates is deposited. Where needed, adhesives may be incorporated in any of these deposition processes, or any adhesive may be applied to the surface of an article separately. Metal nanoparticle agglomerates may become adhered to the surface of an article during its manufacture process, or the metal nanoparticle agglomerates may be applied to the surface of an article following its manufacturing process and subsequently become adhered thereto. As discussed below, metal nanoparticle agglomerates may become at least partially embedded in a polymer surface above the polymer's softening point. In still another approach, metal nanoparticle agglomerates may become adhered to a metal surface by a "burn in" approach by heating the metal nanoparticle agglomerates on the surface for a few minutes at about 200-240°C to promote fusion with a metal surface. Those chosen manner in which the metal nanoparticle agglomerates become adhered to a surface may be determined, at least in part, by the type of surface being coated. Surface types that may be coated with metal nanoparticle agglomerates in the disclosure herein include, for example, glass, metal, ceramics, polymers, cement, textiles, and the like.

Metal nanoparticles and their agglomerates may undergo ready adherence to a range of surface types once deposited in small droplet form thereon. A surfactant coating remains intact upon the surface of metal nanoparticles within metal nanoparticle agglomerates, if the metal nanoparticle agglomerates are suitably bound to the surface of an article via an adhesive layer. Provided the surfactant coating remains intact, the metal nanoparticles may remain unfused with each other and protected from oxidation, such that they may be gradually released from the metal nanoparticle agglomerates and inhibit formation and proliferation of a biofilm.

Spray formulations comprising metal nanoparticle agglomerates may comprise an aerosolizable fluid medium in which an aerosol or similar droplets comprising metal nanoparticle agglomerates may be generated. The aerosolizable fluid medium may comprise a gas or highly volatile liquid at room temperature and pressure, such that metal nanoparticle agglomerates may be rapidly deposited upon the surface of an article, with the surface being obtained in dry form essentially immediately or soon after spraying the metal nanoparticle agglomerates thereon. Suitable spray formulations may also utilize an aerosol propellant or a volatile solvent for spray pumping applications. Aerosol propellants may be particularly desirable due to their essentially instantaneous evaporation once discharged from their source, such as a spray can. Depending on the aerosolizable fluid medium used and the technique for application thereof, the aerosolized droplet size may range from about 10-150 microns for aerosol sprays and from about 150-400 microns for mechanically pumped sprays. The aerosolized droplets are easily directed to a specified location and do not linger overly long in air due to their high density before settling on a surface to deposit metal nanoparticle agglomerates thereon. Additional details regarding suitable spray formulations of various types are also provided below.

As used herein, the term "metal nanoparticles" refers to metal particles that are about 250 nm or less in size, particularly about 200 nm or less in size, or about 150 nm or less in size, without particular reference to the shape of the metal particles. Copper nanoparticles are metal nanoparticles comprising predominantly copper, optionally with an oxide coating wholly or partially covering the surface of the copper nanoparticles. Likewise, silver nanoparticles are metal nanoparticles comprising predominantly silver, optionally with an oxide coating wholly or partially covering the surface of the silver nanoparticles. The term "metal nanoparticle" broadly refers herein to any metallic structure having at least one dimension of 250 nm or less, particularly about 200 nm or less or about 150 nm or less, and includes other structures that are not substantially spherical in nature, such as metal platelets/disks, metal nanowires, or the like. Other metal nanostructures may be used in addition to or as alternatives to spherical or substantially spherical metal nanoparticles, or agglomerates thereof, in the disclosure herein. The metal nanoparticles or similar nanostructures may also feature a surfactant coating, which may provide protection against oxidation, prevent fusion of the metal nanoparticles from taking place, and aid in forming metal nanoparticle agglomerates in some instances. The surfactant coating may or may not be removed or lost as the metal nanoparticles become adhered to a surface. If the surfactant coating is removed, the metal nanoparticles may or may not become at least partially fused to one another, depending on conditions.

Metal nanoparticle agglomerates result when metal nanoparticles associate together but individual metal nanoparticles are still identifiable. The term "metal nanoparticle agglomerates" and equivalent grammatical forms thereof refers to a grouping of metal nanoparticles having at least one dimension ranging from about 0.1 to about 35 microns in size, particularly about 0.1 to about 15 microns in size. Individual metal nanoparticles within a metal nanoparticle agglomerate may reside within the size ranges indicated above and elsewhere herein, and the individual metal nanoparticles may be associated with one another through non-covalent, covalent, or metallic bonding interactions. The term "associated" refers to any type of bonding force that maintains a grouping of metal nanoparticles together, such as van der Waals forces and mechanical entanglement of long-chain surfactants, for example. The bonding force may be overcome to produce individual metal nanoparticles in some instances.

The terms "consolidate," "consolidation" and other variants thereof are used interchangeably herein with the terms "fuse," "fusion" and other variants thereof.

Once a surfactant coating has been lost from the surface of metal nanoparticles, as discussed further below, surface oxidation of the metal nanoparticles may also lead to formation of reactive and potentially mobile salt compounds in combination with the metal nanoparticles upon the surface of an article. Such salts may include, for example, chlorides, bisulfites and bicarbonates. Chlorides, for example, may result from chloride ions in sweat or other bodily fluids. Formation of such salts may be particularly prevalent upon exposure of the metal nanoparticles to a moist environment, as specified for a bicarbonate salt in Reaction 1 below. Dry conditions, in contrast, may favor formation of at least a partial oxide coating upon the surface of the metal nanoparticles (Reaction 2).

Cu + ½ O₂ + H₂O + 2 CO₂ → Cu(HCO₃)₂ (Reaction 1)

Cu + ½ O₂ → Cu₂O (Reaction 2)

The salts may be surfactant-stabilized salt complexes comprising one or more surfactants (e.g., one or more amine surfactants in the case of copper nanoparticles) and sufficient salt anions to achieve charge balance. Charge balancing anions may include, for example, halogen, particularly chloride; bisulfite; bicarbonate; lactate; or the like. The charge balancing anions are relatively labile and may be released to generate open coordination sites for binding DNA, proteins, or like biomolecules. The surfactant-stabilized salt complexes may be relatively mobile upon the surface of an article, even when bound within or upon an adhesive, and provide a higher effective coverage of metal nanoparticles thereupon compared to if they remained fully fixed in place.

In addition to salt compounds or surfactant-stabilized forms thereof formed *in situ* during use, metal salts or surfactant-stabilized forms thereof may be combined with metal nanoparticles or metal nanoparticle agglomerates prior to deposition of the metal nanoparticle agglomerates upon a surface subject to biofilm formation or proliferation. Any of the preceding counteranion forms of the metal salts may be utilized in the disclosure herein. Surprisingly, metal salts or surfactant-stabilized forms thereof may themselves aid in killing or inactivating viruses or bacteria upon release from metal nanoparticle agglomerates. When present, the added metal salt compounds may be present at a ratio ranging from about 1 to about 0.001, or about 0.01 to about 0.001, or about 1 to about 0.1, or about 0.1 to about 0.001, each on a weight basis with respect to the metal nanoparticles. The added metal salt compounds may also be deposited upon a surface separately from the metal nanoparticles or metal nanoparticle agglomerates, such as by forming a solution of metal salt in a solvent such as an alcohol or acetone, for example, which may be contacted with the surface through spraying, dip coating, or a similar application technique. The concentration of metal salt in a spray or dip coating formulation may range from about 0.5 ppm to about 50 ppm. The coating density of the added metal salt upon a fabric or polymer film may range from about 0.0016 mg/cm² to about 0.078 mg/cm² (about 0.01 to about 0.5 mg/in²) or 0.0016 mg/cm² to 0.016 mg/cm² (about 0.01 mg/in² to about 0.1 mg/in²).

In some embodiments, added metal salt compounds may be associated with amine, sulfur, or phosphate functional groups upon a functionalized polymer. The metal salt compounds may be associated with the functional groups by a metal-ligand bond (e.g., in the case of an amine-functionalized polymer) or through salt formation (e.g., in the case of a sulfonic acid-functionalized polymer). Thiol-functionalized polymers or sulfide-functionalized polymers may also coordinate added metal salt compounds. Disulfide-crosslinked polymers, in the presence of a suitable reducing agent, may similarly coordinate added metal salt compounds. Reactions 4-6 below show some of the transformations that added CuCl₂ may undergo (R is a polymer chain).

R-SO₃H + CuCl₂ → R-SO₃-CuCl + HCl (Reaction 4)

R-NH₂ + CuCl₂ → R-NH₂CuCl₂ (Reaction 5)

R₂S + CuCl₂ → R₂SCuCl₂ (Reaction 6)

Before discussing more particular aspects of the present disclosure in further detail, additional brief description of metal nanoparticles and their processing conditions, particularly silver or copper nanoparticles, will first be provided. Metal nanoparticles exhibit a number of properties that can differ significantly from those of the corresponding bulk metal. One property of metal nanoparticles that can be of particular importance for processing is nanoparticle fusion (consolidation) that occurs at the metal nanoparticles' fusion temperature. As used herein, the term "fusion temperature" refers to the temperature at which a metal nanoparticle liquefies, thereby giving the appearance of melting. At or above the fusion temperature, consolidation with other metal nanoparticles may readily take place. As used herein, the terms "fusion" and "consolidation" synonymously refer to the coalescence or partial coalescence of metal nanoparticles with one another to form a larger mass. Metal nanoparticles within a metal nanoparticle agglomerate may undergo fusion with one another or individual metal nanoparticles may become fused as well, thereby forming a network of at least partially fused metal nanoparticles in either case. In some embodiments described herein, at least a portion of the metal nanoparticles in the metal nanoparticle agglomerates remain unfused with one another when adhered to a surface of an article.

Advantageously and surprisingly, metal nanoparticles, such as copper nanoparticles and/or silver nanoparticles, for example, can become adhered to various types of surfaces even well below their fusion temperature, thereby allowing surface bonding to take place, as discussed further herein. Depending on the density at which the metal nanoparticles within metal nanoparticle agglomerates are loaded onto a surface susceptible to biofilm formation and the temperature at which the metal nanoparticles are processed thereon, individual metal nanoparticles may or may not be further fused together when adhered thereto. Desirably, the metal nanoparticles may remain at least partially unfused, preferably substantially unfused, to facilitate time-release of metal nanoparticles and metal nanoparticle clusters (smaller metal nanoparticle agglomerates) from larger metal nanoparticle agglomerates upon an article's surface, but not in a form such that they are substantially released in free form *in vivo.* Alternately or in addition, metal ions and/or metal ion clusters may be released from the metal nanoparticle agglomerates upon the surface. Oxidized forms of the metal nanoparticles may be released as well. When applying metal nanoparticles and their agglomerates to the surface of an article, further heating may or may not be performed, depending upon the extent of metal nanoparticle fusion that is desired. Preferably, no heating is performed so that the metal nanoparticles remain substantially unfused upon the surface of the article in the form of metal nanoparticle agglomerates. Alternately, the temperature may remain sufficiently low that the metal nanoparticles do not become fused together while being further processed upon the surface.

Upon decreasing in size, particularly below about 20 nm in equivalent spherical diameter, the temperature at which metal nanoparticles liquefy drops dramatically from that of the corresponding bulk metal. For example, copper nanoparticles having a size of about 20 nm or less can have fusion temperatures of about 220°C or below, or about 200°C or below, or even about 175°C or below in comparison to bulk copper's melting point of 1083°C. Silver nanoparticles may similarly display a significant deviation from the melting point of bulk silver below a nanoparticle size of about 20 nm. Thus, the consolidation of metal nanoparticles taking place at the fusion temperature as a consequence of the high surface energy can allow structures containing bulk metal to be fabricated at significantly lower processing temperatures than when working directly with the bulk metal itself as a starting material. Although metal nanoparticles having a size of about 20 nm or under may be advantageous in some situations, even larger metal nanoparticles may be suitable in the disclosure herein when utilized within metal nanoparticle agglomerates. More specifically, by using metal nanoparticles or agglomerates thereof, bulk metal may be dispersed upon various surfaces susceptible to biofilm formation that would otherwise be thermally incompatible with the processing temperatures required to introduce molten metal thereon. Once deposited upon a surface, metal nanoparticles or agglomerates thereof may become strongly adhered to the surface even without being raised above the fusion temperature and forming bulk metal, as described further herein. The small particle sizes of the metal nanoparticles and their agglomerates may promote ready dispersion within an aerosolizable fluid medium for processing into spray formulations or similar coating formulations for application upon a surface.

A number of scalable processes for producing bulk quantities of metal nanoparticles in a targeted size range have been developed. Most typically, such processes for producing metal nanoparticles take place by reducing a metal precursor in the presence of one or more surfactants. The as-isolated metal nanoparticles may have a surfactant coating thereon and be isolated as a plurality of metal nanoparticle agglomerates. The agglomerates may be broken apart, while retaining the surfactant coating, or the agglomerates may be used directly without further processing. Particularly advantageous metal nanoparticle sizes that may present within metal nanoparticle agglomerates in the disclosure herein may range from about 50 nm to about 250 nm in size, optionally in combination with at least some metal nanoparticles that are about 20 nm or under in size. The agglomerates, by virtue of having a range of particle sizes, may convey a time-release profile for providing individual metal nanoparticles, smaller metal nanoparticle agglomerates, metal ions, and/or metal ion clusters upon the surface of an article susceptible to biofilm formation when utilized in the disclosure herein. While present, the surfactants upon the metal nanoparticles may facilitate surface adhesion through van der Waals interactions. After surfactant loss takes place, a highly active metal form to promote surface adherence may be realized.

Metal nanoparticle agglomerates in the disclosure herein may be of an advantageous size range to facilitate dispensation via spraying or other coating processes and to promote retention upon a surface, such an agglomerate size ranging from about 1 micron to about 35 microns or about 1 micron to about 15 microns. The metal nanoparticles or agglomerates thereof can be isolated and purified from a reaction mixture by common isolation techniques and processed into a suitable spray formulation or similar coating formulation for surface dispensation. The surfactant coating upon the metal nanoparticles may be removed through gentle heating, gas flow, and/or vacuum (any pressure below atmospheric pressure) once the metal nanoparticles or agglomerates thereof have been deposited upon a surface of an article, thereby affording a much higher surface energy and a commensurate increase in reactivity. Alternately, the surfactant coating may be lost upon extended contact with a surface without undergoing additional heating or other processing, with adherence to the surface occurring following surfactant loss. The surfactant coating may remain for at least some period of time upon the surface of an article, such that the metal nanoparticles are retained as individuals thereon without becoming fused to each other or being converted into a highly active form while still being present within the metal nanoparticle agglomerates. Once the surfactant coating has been removed or lost, the high surface energy of the metal nanoparticles may facilitate adherence of the metal nanoparticles to the surface through chemical bonding. The metal nanoparticles may become fused together during this process or they may remain unfused, preferably unfused for purposes of inhibiting biofilm growth or proliferation. Thus, metal nanoparticle agglomerates may retain individual, unfused metal nanoparticles as the surfactant coating is removed. Alternately or in addition, the metal nanoparticles may become at least partially oxidized as a surfactant coating is lost. At least some surface adhesion may also be realized without the surfactant coating being removed, particularly when the metal nanoparticle agglomerates are present in combination with an adhesive.

Agglomerates having a range of sizes, such as those within a range of about 1 micron to about 35 microns, or about 1 micron to about 15 microns, or about 1 micron to about 5 microns, may be advantageous in terms of their ability to be dispensed through aerosol formation or sprayed droplets or via introduction into other types of coating formulations. Agglomerates of metal nanoparticles having different agglomerate sizes may release metal nanoparticles and even smaller clusters of metal nanoparticles at different rates upon the surface of an article to become infiltrated within a biofilm, thereby affording a biocidal environment for microbes therein, but without being substantially released *in vivo.* As metal nanoparticles or similar active metal species are released from the metal nanoparticle agglomerates at different rates, the metal nanoparticles and/or metal ions or small clusters thereof may migrate over a surface to afford biocidal coverage that is more complete than the actual coverage density of the metal nanoparticle agglomerates upon the surface. Thus, the metal nanoparticles may attack bacteria in contact with any portion of the surface, as long as metal nanoparticle agglomerates are sufficiently close by to provide metal nanoparticles to the surface in a bound form, either directly bound to the surface and/or further constrained with an adhesive. Biofilm formation and proliferation may be inhibited in either case. By differentially releasing metal nanoparticles or other active metal species from metal nanoparticle agglomerates having a range of sizes upon an article's surface, a time-release profile of active metal species may be realized to afford a prolonged activity against biofilm formation and proliferation. Thus, the metal nanoparticle agglomerates and their binding properties may be tailored to provide activity against biofilm formation and proliferation that may be retained over several days, such as at least about 3 days, or at least about 5 days, or at least about 7 days, or at least about 10 days, or at least about 14 days, or at least about 21 days, or at least about 30 days. An adhesive in contact with the metal nanoparticle agglomerates may further facilitate a time-release profile of active metal species upon the surface of an article while precluding release of free metal nanoparticles from the base substrate. Suitable adhesives are not considered to be particularly limited and are specified in more detail below. For *in vivo* applications, biologically compatible adhesives may be preferred.

Any suitable technique can be employed for forming the metal nanoparticles used in the disclosure herein. Particularly facile metal nanoparticle fabrication techniques, particularly for copper nanoparticles, are described in U.S. Pat. Nos. 7,736,414, 8,105,414, 8,192,866, 8,486,305, 8,834,747, 9,005,483, 9,095,898, and 9,700,940. Similar procedures may be used for synthesizing other types of metal nanoparticles. As described therein, metal nanoparticles can be fabricated in a narrow size range by reduction of a metal salt in a solvent in the presence of a suitable surfactant system, which can include one or more different surfactants. Further description of suitable surfactant systems follows below. Tailoring of the surfactant system, the reaction concentration, temperature, and like factors may determine the size range of metal nanoparticles that are obtained from a metal nanoparticle synthesis. Without being bound by any theory or mechanism, it is believed that the surfactant system can mediate the nucleation and growth of the metal nanoparticles, limit surface oxidation of the metal nanoparticles while the surfactant system is adhered thereto, and/or inhibit metal nanoparticles from extensively aggregating with one another prior to being at least partially fused together. As noted above, small agglomerates of metal nanoparticles may be formed in many instances and used in the disclosure herein. Suitable organic solvents for solubilizing metal salts and forming metal nanoparticles can include, for example, formamide, N,N-dimethylformamide, dimethyl sulfoxide, dimethylpropylene urea, hexamethylphosphoramide, tetrahydrofuran, glyme, diglyme, triglyme, tetraglyme, proglyme, or polyglyme. Reducing agents suitable for reducing metal salts and promoting the formation of metal nanoparticles can include, for example, an alkali metal in the presence of a suitable catalyst (*e.g.*, lithium naphthalide, sodium naphthalide, or potassium naphthalide) or borohydride reducing agents (*e.g*., sodium borohydride, lithium borohydride, potassium borohydride, or tetraalkylammonium borohydrides). In non-limiting examples, reduction of the metal salt to form metal nanoparticles and agglomerates thereof may take place under substantially anhydrous conditions in a suitable organic solvent.

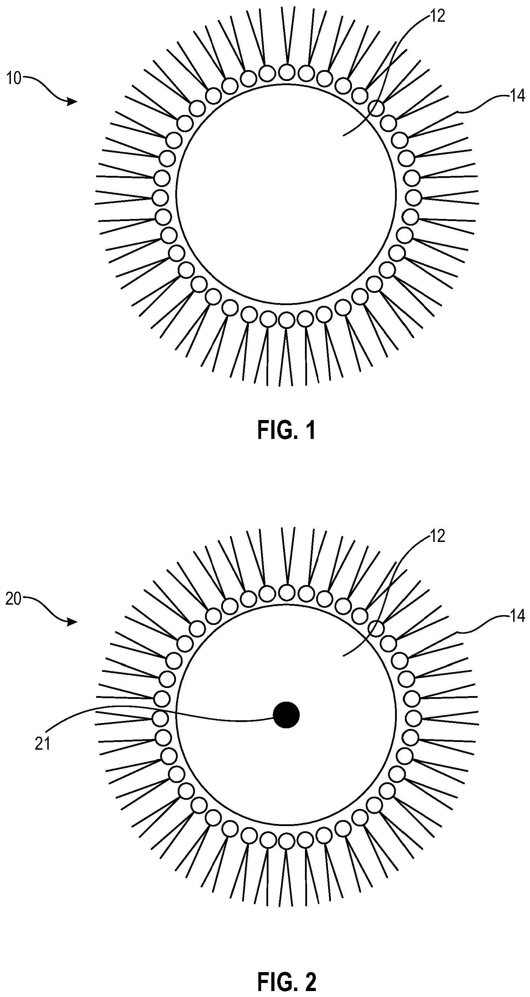

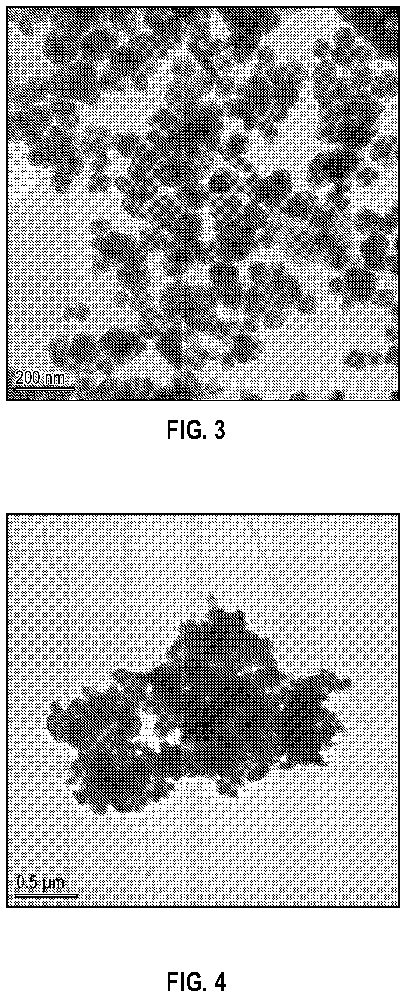

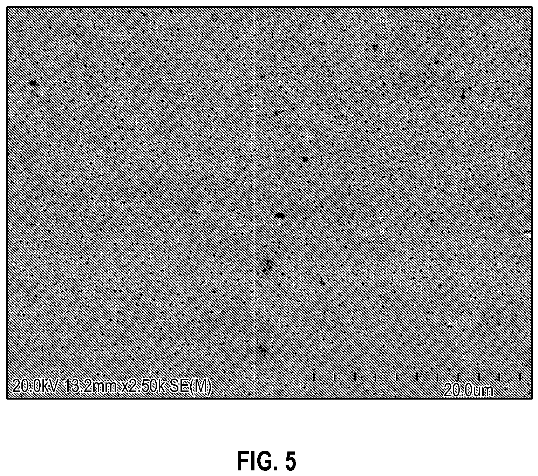

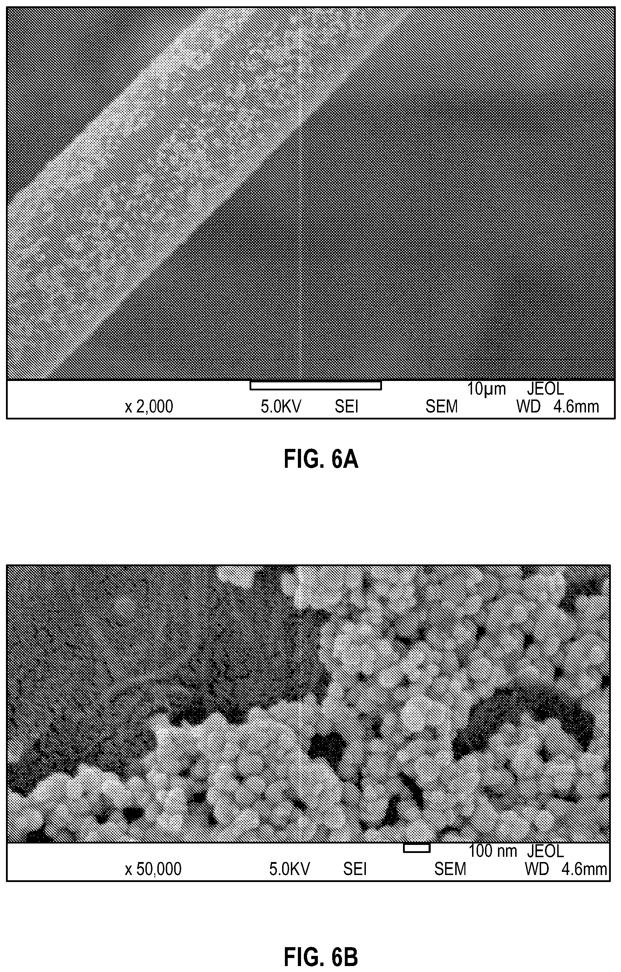

FIGS. 1 and 2 show diagrams of presumed structures of metal nanoparticles having a surfactant coating thereon. As shown in FIG. 1, metal nanoparticle 10 includes metallic core 12 and surfactant layer 14 overcoating metallic core 12. Surfactant layer 14 can contain any combination of surfactants, as described in more detail below. Metal nanoparticle 20, shown in FIG. 2, is similar to that depicted in FIG. 1, except metallic core 12 is grown about nucleus 21. Because nucleus 21 is buried deep within metallic core 12 in metal nanoparticle 20 and is very small in size, it is not believed to significantly affect the overall nanoparticle properties. Nucleus 21 may comprise a salt or a metal, wherein the metal may be the same as or different than that of metallic core 12. In some embodiments, the nanoparticles can have an amorphous morphology. FIGS. 1 and 2 may be representative of the microscopic structure of copper or silver nanoparticles suitable for use in the disclosure herein. FIG. 3 shows an illustrative SEM image of substantially individual copper nanoparticles. FIG. 4 shows an illustrative SEM image of an agglomerate of copper nanoparticles, which may be used in the disclosure herein. FIG. 5 shows an illustrative SEM image of a copper nanoparticle network obtained after fusion of a plurality of copper nanoparticles to each other. FIGS. 6A and 6B show illustrative SEM images of agglomerates of copper nanoparticles adhered to textile fibers. The agglomerates of copper nanoparticles are robustly adhered to the textile fibers but do not undergo fusion with one another. The bonding to textile fibers may be representative of the bonding of metal nanoparticle agglomerates to a surface of an article in accordance with the present disclosure.

As discussed above, the metal nanoparticles have a surfactant coating containing one or more surfactants upon their surface. The surfactant coating can be formed on the metal nanoparticles during their synthesis. Formation of a surfactant coating upon metal nanoparticles during their syntheses can desirably limit the ability of the metal nanoparticles to fuse to one another prematurely, limit agglomeration of the metal nanoparticles to a desired extent or agglomerate size, and promote the formation of a population of metal nanoparticles having a narrow size distribution. At least partial loss of the surfactant coating may occur upon heating the metal nanoparticles up to the fusion temperature, including at least some surfactant loss well below the fusion temperature for low-boiling surfactants. Surfactant loss may be further promoted by flowing gas and/or application of vacuum (reduced pressure), as desired, even below the fusion temperature. At least some surfactant loss may occur at room temperature and ambient pressure conditions in some instances when the metal nanoparticles are deposited upon a surface. Following surfactant loss, fusion of the metal nanoparticles may take place above the fusion temperature. If the uncoated metal nanoparticles are not heated above the fusion temperature and remain unfused, an uncoated metal nanoparticle having a high surface energy may be obtained. Surface oxidation may take place in these circumstances and others. The high surface energy may promote adherence of the metal nanoparticles to the surface and provide a highly reactive metal form for inhibiting formation of a biofilm. The metal nanoparticles may become adhered to a surface of an article even below the fusion temperature once the surfactant coating has been removed. When heated above the fusion temperature, nanoparticle fusion may take place in combination with the metal nanoparticles becoming adhered to the surface.

Various types of metal nanoparticles may be synthesized by metal reduction in the presence of one or more suitable surfactants, such as copper nanoparticles or silver nanoparticles. Copper and/or silver can be particularly desirable metals due to their ability to promote killing or inactivation of bacteria upon a surface, thereby inhibiting biofilm formation. Significantly higher biocidal activity for both of these metals may be realized when utilizing metal nanoparticles compared to that obtained for a bulk metal surface. Copper may also be particularly advantageous due to its low cost. Zinc can similarly display biocidal activity against bacteria. Zinc may also be used in combination with copper. NiO and TiO₂ may be used similarly in this respect. Nanoparticle forms of Zn, Ni, Co, and Ti may be utilized as well, in combination with copper nanoparticles at least partially oxidized variants of copper nanoparticles. In accordance with the invention, the metal nanoparticle agglomerates comprise copper nanoparticles and Cu₂O is present in combination with the copper nanoparticles.

In accordance with the invention, the metal nanoparticle agglomerates comprise a plurality of metal nanoparticles having a surfactant coating thereon. The surfactant system present within the metal nanoparticles includes one or more surfactants. The differing properties of various surfactants can be used to tailor the properties of the metal nanoparticles. Factors that can be taken into account when selecting a surfactant or combination of surfactants for inclusion upon the metal nanoparticles can include, for example, ease of surfactant dissipation from the metal nanoparticles during or prior to nanoparticle fusion, nucleation and growth rates of the metal nanoparticles to impact the nanoparticle size, the metal component of the metal nanoparticles, and the like. Main group metals, for example, may require different surfactants than do transition metals when forming metal nanoparticles.

In some embodiments, an amine surfactant or combination of amine surfactants, particularly aliphatic amines, can be present upon the metal nanoparticles. Amine surfactants can be particularly desirable for use in conjunction with copper nanoparticles or silver nanoparticles due to their good affinity for these transition metals. In some embodiments, two amine surfactants can be used in combination with one another. In other embodiments, three amine surfactants can be used in combination with one another. In more specific embodiments, a primary amine, a secondary amine, and a diamine chelating agent can be used in combination with one another. In still more specific embodiments, the three amine surfactants can include a long chain primary amine, a secondary amine, and a diamine having at least one tertiary alkyl group nitrogen substituent. This combination of surfactants may be particularly effective for producing metal nanoparticles within a desired size range. Further disclosure regarding suitable amine surfactants follows hereinafter.

In some embodiments, the surfactant system can include a primary alkylamine. In some embodiments, the primary alkylamine can be a C₂-C₁₈ alkylamine. In some embodiments, the primary alkylamine can be a C₇-C₁₀ alkylamine. In other embodiments, a C₅-C₆ primary alkylamine can also be used. Without being bound by any theory or mechanism, the exact size of the primary alkylamine can be balanced between being long enough to provide an effective inverse micelle structure during synthesis versus having ready volatility and/or ease of handling during nanoparticle consolidation. For example, primary alkylamines with more than 18 carbons can also be suitable for use in the present embodiments, but they can be more difficult to handle because of their waxy character. C₇-C₁₀ primary alkylamines, in particular, can represent a good balance of desired properties for ease of use.

In some embodiments, the C₂-C₁₈ primary alkylamine can be n-hexylamine, n-heptylamine, n-octylamine, n-nonylamine, or n-decylamine, for example. While these are all straight chain primary alkylamines, branched chain primary alkylamines can also be used in other embodiments. For example, branched chain primary alkylamines such as, for example, 7-methyloctylamine, 2-methyloctylamine, or 7-methylnonylamine can be used. In some embodiments, such branched chain primary alkylamines can be sterically hindered where they are attached to the amine nitrogen atom. Non-limiting examples of such sterically hindered primary alkylamines can include, for example, t-octylamine, 2-methylpentan-2-amine, 2-methylhexan-2-amine, 2-methylheptan-2-amine, 3-ethyloctan-3-amine, 3-ethylheptan-3-amine, 3-ethylhexan-3-amine, and the like. Additional branching can also be present. Without being bound by any theory or mechanism, it is believed that primary alkylamines can serve as ligands in the metal coordination sphere but be readily dissociable therefrom during metal nanoparticle consolidation.

In some embodiments, the surfactant system can include a secondary amine. Secondary amines suitable for forming metal nanoparticles can include normal, branched, or cyclic C₄-C₁₂ alkyl groups bound to the amine nitrogen atom. In some embodiments, the branching can occur on a carbon atom bound to the amine nitrogen atom, thereby producing significant steric encumbrance at the nitrogen atom. Suitable secondary amines can include, without limitation, dihexylamine, diisobutylamine, di-t-butylamine, dineopentylamine, di-t-pentylamine, dicyclopentylamine, dicyclohexylamine, and the like. Secondary amines outside the C₄-C₁₂ range can also be used, but such secondary amines can have undesirable physical properties such as low boiling points or waxy consistencies that can complicate their handling.

In some embodiments, the surfactant system can include a chelating agent, particularly a diamine chelating agent. In some embodiments, one or both of the nitrogen atoms of the diamine chelating agent can be substituted with one or two alkyl groups. When two alkyl groups are present on the same nitrogen atom, they can be the same or different. Further, when both nitrogen atoms are substituted, the same or different alkyl groups can be present. In some embodiments, the alkyl groups can be C₁-C₆ alkyl groups. In other embodiments, the alkyl groups can be C₁-C₄ alkyl groups or C₃-C₆ alkyl groups. In some embodiments, C₃ or higher alkyl groups can be straight or have branched chains. In some embodiments, C₃ or higher alkyl groups can be cyclic. Without being bound by any theory or mechanism, it is believed that diamine chelating agents can facilitate metal nanoparticle formation by promoting nanoparticle nucleation.

In some embodiments, suitable diamine chelating agents can include N,N'-dialkylethylenediamines, particularly C₁-C₄ N,N'-dialkylethylenediamines. The corresponding methylenediamine, propylenediamine, butylenediamine, pentylenediamine or hexylenediamine derivatives can also be used. The alkyl groups can be the same or different. C₁-C₄ alkyl groups that can be present include, for example, methyl, ethyl, propyl, and butyl groups, or branched alkyl groups such as isopropyl, isobutyl, s-butyl, and t-butyl groups. Illustrative N,N'-dialkylethylenediamines that can be suitable for inclusion upon metal nanoparticles include, for example, N,N'-di-t-butylethylenediamine, N,N'-diisopropylethylenediamine, and the like.

In some embodiments, suitable diamine chelating agents can include N,N,N',N'-tetraalkylethylenediamines, particularly C₁-C₄ N,N,N',N'-tetraalkylethylenediamines. The corresponding methylenediamine, propylenediamine, butylenediamine, pentylenediamine or hexylenediamine derivatives can also be used. The alkyl groups can again be the same or different and include those mentioned above. Illustrative N,N,N',N'-tetraalkylethylenediamines that can be suitable for use in forming metal nanoparticles include, for example, N,N,N',N'-tetramethylethylenediamine, N,N,N',N'-tetraethylethylenediamine, and the like.

Surfactants other than aliphatic amines can also be present in the surfactant system. In this regard, suitable surfactants can include, for example, pyridines, aromatic amines, phosphines, thiols, or any combination thereof. These surfactants can be used in combination with an aliphatic amine, including those described above, or they can be used in a surfactant system in which an aliphatic amine is not present. Further disclosure regarding suitable pyridines, aromatic amines, phosphines, and thiols follows below.

Suitable aromatic amines can have a formula of ArNR¹R², where Ar is a substituted or unsubstituted aryl group and R¹ and R² are the same or different. R¹ and R² can be independently selected from H or an alkyl or aryl group containing from 1 to about 16 carbon atoms. Illustrative aromatic amines that can be suitable for use in forming metal nanoparticles include, for example, aniline, toluidine, anisidine, N,N-dimethylaniline, N,N-diethylaniline, and the like. Other aromatic amines that can be used in conjunction with metal nanoparticles can be envisioned by one having ordinary skill in the art.

Suitable pyridines can include both pyridine and its derivatives. Illustrative pyridines that can be suitable for inclusion upon metal nanoparticles include, for example, pyridine, 2-methylpyridine, 2,6-dimethylpyridine, collidine, pyridazine, and the like. Chelating pyridines such as bipyridyl chelating agents may also be used. Other pyridines that can be used in conjunction with metal nanoparticles can be envisioned by one having ordinary skill in the art.

Suitable phosphines can have a formula of PR₃, where R is an alkyl or aryl group containing from 1 to about 16 carbon atoms. The alkyl or aryl groups attached to the phosphorus center can be the same or different. Illustrative phosphines that can be present upon metal nanoparticles include, for example, trimethylphosphine, triethylphosphine, tributylphosphine, tri-t-butylphosphine, trioctylphosphine, triphenylphosphine, and the like. Phosphine oxides can also be used in a like manner. In some embodiments, surfactants that contain two or more phosphine groups configured for forming a chelate ring can also be used. Illustrative chelating phosphines can include 1,2-bisphosphines, 1,3-bisphosphines, and bis-phosphines such as BINAP, for example. Other phosphines that can be used in conjunction with metal nanoparticles can be envisioned by one having ordinary skill in the art.

Suitable thiols can have a formula of RSH, where R is an alkyl or aryl group having from about 4 to about 16 carbon atoms. Illustrative thiols that can be present upon metal nanoparticles include, for example, butanethiol, 2-methyl-2-propanethiol, hexanethiol, octanethiol, benzenethiol, and the like. In some embodiments, surfactants that contain two or more thiol groups configured for forming a chelate ring can also be used. Illustrative chelating thiols can include, for example, 1,2-dithiols (*e.g*., 1,2-ethanethiol) and 1,3-dithiols (*e.g.,* 1,3-propanethiol). Other thiols that can be used in conjunction with metal nanoparticles can be envisioned by one having ordinary skill in the art.

As mentioned above, a distinguishing feature of metal nanoparticles is their high surface energy, particularly after removal of a surfactant coating therefrom, which may promote their adherence to the surface of an article in a form suitable for providing a highly active metal species upon the surface. A surfactant associated with the metal nanoparticles may further facilitate surface adhesion, especially prior to surfactant loss and formation of uncoated metal nanoparticles having a high surface energy. That is, the surfactant coating may initially hold metal nanoparticle agglomerates in place (*e.g*., through van der Waals forces) until a high surface energy state has been attained to promote more robust adherence and retention of the metal nanoparticles upon a surface. Robust adherence to a surface may still be realized even with the surfactant coating intact, however, particularly when an adhesive is used. In accordance with the present invention, the metal nanoparticle agglomerates comprise a plurality of metal nanoparticles having a surfactant coating thereon.

The loading of metal nanoparticles as metal nanoparticle agglomerates upon the surface of an article in need of protection against biofilm formation may include a coverage density ranging from about 0.016 mg/cm² (about 0.1 mg/in²) to about 1.6 mg/cm² (about 10 mg/in²), or about 0.078 mg/cm² (about 0.5 mg/in²) to 0.78 mg/cm² (about 5 mg/in²), or about 0.16 mg/cm² (about 1 mg/in²) to about 0.32 mg/cm² (about 2 mg/in²) or about 0.078 mg/cm² (about 0.5 mg/in²) to 0.48 mg/cm² (about 3 mg/in²), or about 0.16 mg/cm² (1 mg/in²) to about 0.78 mg/cm² (about 5 mg/in²). The coverage of metal nanoparticles as metal nanoparticle agglomerates upon the surface may range from about 5 % to about 95 % by area, or about 50 % to about 99 % by area, or about 60 % to 95 % by area. Even coverage densities as low as 3-5 % by area may be effective for inhibiting biofilm formation or proliferation in the disclosure herein.

As-produced metal nanoparticles are usually produced in the form of agglomerates, which are then broken apart into individual surfactant-coated metal nanoparticles in order to promote use in various applications. Surprisingly, in the disclosure herein, the as-produced agglomerates, such as those residing in a 0.1-35 micron size range, particularly a 0.1-15 micron size range or a 0.1-5 micron size range, may facilitate ready dispensation and adherence upon a surface and biocidal activity thereupon. Even larger metal nanoparticle agglomerates, up to about 100 microns in size, may be applicable in some instances and effective for dispensation upon a surface in various manners. Such agglomerates may change their shape as they adhere to a surface, while remaining bound to each other in a "colony." Within the metal nanoparticle agglomerates, recognizable sub-structures may be present prior to nanoparticle fusion such as, but not limited to, 10-50 nm thick platelets having a width of about 100-250 nm, 1-5 nm thick platelets having a width of about 30-50 nm, 100-250 nm wide spheres, metal nanowires, the like, or any combination thereof. The sub-structures may have any shape such as square, triangular, rectangular, multifaceted, round, and ovular, and crystalline, and/or non-crystalline morphologies. Elongate structures, such as metal nanowires, may have an aspect ratio of at least about 10 or at least about 25, for example. Copper nanoparticles and/or silver nanoparticles may also be combined with pre-made nanowires (*e.g.*, copper nanowires or silver nanowires) and deposited upon a surface as well. Oxide forms of copper or silver, such as Cu₂O or AgO, may also be present after deposition upon a surface subject to biofilm formation. Zinc, nickel, titanium, or cobalt, particularly in the form of nanoparticles or a salt form or metal oxide form thereof, may be present as well.

When present at the foregoing coverages and coverage densities upon the surface of an article, the metal nanoparticles and their agglomerates may effectively inactivate bacteria, oftentimes more effectively than does a bulk metal surface comprising the same metal. For example, copper nanoparticles adhered to a surface and retaining their nanoparticulate form may afford inactivation within as little as 5 minutes, sometimes as little as 30 seconds, or even as little as 5-10 seconds. Up to 100 % kill rates or inactivation rates may be realized. Bulk copper surfaces, in contrast, may take several hours to reach the same level of inactivation.

Accordingly, biofilm-resistant articles adapted for preventing or limiting biofilm formation comprise a surface adapted to contact a liquid medium at least periodically, including submersion in the liquid medium in some instances, and metal nanoparticles agglomerates adhered as a coating to at least a portion of the surface, wherein the metal nanoparticles or agglomerates thereof are present in an effective amount to at least partially inhibit biofilm formation or proliferation. The metal nanoparticle agglomerates comprise a plurality of metal nanoparticles having a surfactant coating thereon. The metal nanoparticles are fused, partially fused, and/or unfused and are associated with one another upon the surface. The metal nanoparticles comprise copper nanoparticles and Cu₂O formed from at least partial oxidation of the copper nanoparticles. In more specific examples, the biofilm-resistant articles may comprise a surface subject to biofilm formation that is adapted to contact a liquid at least periodically, particularly a liquid medium that may encourage biofilm formation (*e.g*., through surface conditioning), and a coating comprising metal nanoparticle agglomerates adhered to at least a portion of the surface. The liquid medium with which the article is in contact at least periodically for promoting biofilm formation may be a fluid capable of forming a conditioning film that may promote biofilm formation upon at least a portion of the surface of the article. Particular examples of articles that may be coated according to the disclosure herein include those that are implantable *in vivo* and/or positionable upon a patient's skin or external tissue and contact a biological fluid capable of promoting biofilm formation, or articles that are at least partially submerged or submersible or experience periodic contact with water, such as in an environmental setting. More specific examples of such articles follow below.

The article having an at least partial coating comprising metal nanoparticle agglomerates thereon may have a surface comprising a material selected from a polymer, a metal, glass, a ceramic, a textile or any combination thereof. Polymer surfaces, in some instances, may have the metal nanoparticle agglomerates at least partially embedded therein if the surface is processed above the melting point or softening temperature of the polymer (in the case of thermoplastic polymers) following deposition of metal nanoparticle agglomerates thereon. Crosslinked polymers may similarly have metal nanoparticle agglomerates embedded in the surface (as well as dispersed throughout the crosslinked polymer) if crosslinking of a suitable monomer takes place in the presence of the metal nanoparticle agglomerates. In still another example, metal nanoparticle agglomerates may be "baked in" to the surface of a metal surface in some instances.

The metal nanoparticles within the metal nanoparticle agglomerates comprise copper nanoparticles, or a combination of copper nanoparticles and silver nanoparticles. Copper nanoparticles in an amount effective to limit biofilm growth and proliferation may include a coverage of about 60 % to about 95 % of a specified portion of a surface by area and a coverage density of about 0.16 mg/cm² (about 1 mg/in²) to about 1.6 mg/cm² (about 10 mg/in²) or about 0.16 mg/cm² (about 1 mg/in²) to about 0.78 mg/cm² (about 5 mg/in²). At least partial oxidation of copper nanoparticles to Cu₂O may be particularly effective in regard to the foregoing. Without being bound by any theory or mechanism, it is believed that Cu(0) may be oxidized to Cu(I) on the surface in a slow process, with further oxidation to Cu(II) taking place rapidly thereafter. The CuO/Cu₂O coating on the surface of copper nanoparticles may provide advantageous biocidal effects against a forming biofilm. When the metal nanoparticle agglomerates contact bacteria potentially able to form a biofilm, hydroxyl radicals and lipid radicals may form, which may disrupt the outer lipid bilayer or protein shell of a bacterium. In addition, copper may bind to heteroatoms (*e.g*., S, N or P) within amino acids, proteins, DNA and/or RNA of bacteria to result in inactivation or killing thereof. Metal penetration within a cell membrane or protein coat may also occur, wherein the metal may inhibit DNA/RNA replication and/or inhibit protein transport.

Combinations of copper nanoparticles and silver nanoparticles may afford particular synergy against biofilms and their associated bacteria that are not remediated adequately with a single metal alone. That is, copper nanoparticles and silver nanoparticles may convey biocidal activity against different types of bacteria, including genetically diverse variants thereof, that may be present in a biofilm. In addition, enhanced activity against a particular type of bacteria may be realized when both copper nanoparticles and silver nanoparticles are present, as compared to copper nanoparticles or silver nanoparticles alone. Without being bound by theory or mechanism, two different types of metal nanoparticles may target different biological pathways and receptors within a given type of bacteria, thereby affording more effective killing or inactivation than is possible with either type of metal nanoparticle alone. The ability to target multiple biological pathways may be particularly advantageous when inhibiting biofilm formation due to the potential exchange of genetic information amongst different bacterial species comprising a particular biofilm. In addition to copper and/or silver nanoparticles or oxidized forms of these metals, Ni, Ti, Co, or Zn nanoparticles, or oxide forms of these metals may be present within a surface coating as well. Conventional biocidal agents, including antibiotic substances, may be present as well.

Metal nanoparticles may be disposed upon the surface of an article from solutions or suspensions of the metal nanoparticles or their agglomerates, which may be formulated with further additives to make the resulting liquid form suitable for dispensation by a given application technique. Particularly suitable liquid forms containing metal nanoparticle agglomerates may be dispensed by spraying, printing, dip coating, pouring, painting, stenciling, or the like. The metal nanoparticles may be adhered to the surface after being deposited thereon or may become adhered at a later time. The metal nanoparticles within the metal nanoparticle agglomerates may remain as individual metal nanoparticles after being adhered to the surface or may become at least partially fused to one another. More desirably, the metal nanoparticles may remain unfused and associated as agglomerates upon the surface of an article in the disclosure herein, wherein the agglomerates are adhered to the surface of the article, with adherence optionally being further promoted with an adhesive.

An adhesive may bind the metal nanoparticles or agglomerates thereof in place to further decrease potential toxicity effects and to promote retention upon the surface of an article. Preferably, an adhesive is present to afford robust adherence of the metal nanoparticle agglomerates to a surface. The adhesive may be a biologically compatible adhesive, which may be desirable for articles destined for *in vivo* implantation or application to an external tissue of a patient. The adhesive may be present upon the surface of an article being coated with the metal nanoparticle agglomerates, or the adhesive may be mixed with the metal nanoparticle agglomerates as they are applied to the surface. As still another option, an adhesive may be applied to the surface of an article after metal nanoparticle agglomerates have been deposited thereon.

Suitable adhesives may include contact and non-contact adhesives. Suitable adhesives will be familiar to one having ordinary skill in the art and include conventional epoxy adhesives, nitrile rubber adhesives, acrylic adhesives, cyanoacrylate adhesives, and the like. Particularly suitable adhesive may be biologically compatible adhesives such as octyl cyanoacrylate, 2-octyl cyanoacrylate, butyl cyanoacrylate, and isobutyl cyanoacrylate. Other examples of suitable adhesives having biocompatibility include, for example, polydioxanone, polyglycolic acid, polylactic acid, and polyglyconate. MAXON, a polyglycolide-trimethylene carbonate used a biodegradable suture adhesive, may represent a particular example. The adhesive may be present at a loading of about 0.0078 mg/cm² (about 0.05 mg/in²) to about 0.078 mg/cm² (about 0.5 mg/in²) or about 0.016 mg/cm² (about 0.1 mg/in²) to about 0.078 mg/cm² (about 0.5 mg/in²) upon the surface of an article. Suitable loadings of the adhesive in a formulation being applied (*e.g*., by painting, dip coating, or spraying) to the surface of an article may include about 0.15 g adhesive/100 g formulation to about 2.75 g adhesive/100 g formulation or about 0.35 g adhesive/100 g formulation to about 2.75 g adhesive/100 g formulation. Coverage of the adhesive upon an article's surface may range from about 50 % to about 100 % by area, or about 60 % to about 90 % by area, or about 75 % to about 95 % by area, or about 90 % to about 99 % by area. A layer thickness of the adhesive upon the surface may be about 300 nm or less, or about 100 nm or less, or about 50 nm or less, such as about 1 nm to about 2 nm, or about 2 nm to about 5 nm, or about 5 nm to about 10 nm, or about 10 nm to about 50 nm, or about 10 nm to about 300 nm. In addition to promoting surface adherence, the adhesive may slow down the production of oxidized metal species and further tailor the time-release profile of individual metal nanoparticles or small agglomerates of metal nanoparticles or various oxidized forms thereof.

Spray formulations suitable for dispensation by pumping or forced pressurization with a gas may exhibit a viscosity value of about 1 cP to about 500 cP, including about 1 cP to about 100 cP. Low viscosity values such as these may facilitate dispensation through spraying promoted by mechanical pumping or forced pressurization. Metal nanoparticle agglomerate loadings within the spray formulations to produce the foregoing viscosity values may range from about 1 wt. % to about 60 wt. %, or about 5 wt. % to about 55 wt. %, or about 10 wt. % to about 45 wt. %, or about 0.5 wt. % to 10 wt. %, or about 10 wt. % to about 35 wt. %, about 1 wt. % to about 35 wt. %, or about 5 wt. % to about 35 wt. %, or about 10 wt. % to about 25 wt. %, or about 0.5 wt. % to 5 wt. %, or about 10 wt. % to about 15 wt. %. Similar loadings of metal nanoparticle agglomerates may be present within formulations adapted for coating the surface of an article by alternative techniques, such as dip coating, painting, stenciling, or the like. Spray formulations configured for deposition using an aerosol propellant may likewise have a similar loading of metal nanoparticle agglomerates.

Spray formulations comprising an aerosol propellant may be suitable for applying metal nanoparticle agglomerates upon the surface of an article according to the disclosure herein. Such spray formulations may similarly comprise metal nanoparticle agglomerates dispersed in a fluid medium comprising at least an aerosol propellant and optionally other solvents to promote metal nanoparticle dispersion therein. Aerosol spray formulations may constitute a particularly desirable form for dispensing the metal nanoparticle agglomerates, since aerosol spray cans are in wide use and are easily manufactured and shipped. Aerosol propellants may afford sprayed droplets ranging from about 10-150 microns in size, whereas mechanically pumped or forced pressurization sprays may have a larger droplet size in a range of about 150-400 microns.

Any conventional aerosol propellant may be utilized in a spray formulation, provided that the metal nanoparticle agglomerates can be effectively dispersed therein, optionally in combination with one or more additional solvents, and ejected from a spray can. Organic and/or inorganic aerosol propellants may be used. Suitable inorganic aerosol propellants may include, for example, nitrous oxide or carbon dioxide. Suitable organic aerosol propellants may include, for example, volatile hydrocarbons (*e.g.,* ethane, propane, butane, or isobutane), dimethyl ether, ethyl methyl ether, hydrofluorocarbons, hydrofluoroolefins, or any combination thereof. Chlorofluorocarbons and similar compounds may also be used as an aerosol propellant, but their use is not preferred due to their ozone-depleting properties. Nevertheless, chlorofluorocarbons may be satisfactory alternatives in situations where other organic aerosolizable fluid media may not be effectively used.

When using an aerosol propellant to promote dispensation of metal nanoparticle agglomerates, the metal nanoparticle agglomerates may be directly combined therewith, or the metal nanoparticles may be dissolved in a secondary fluid medium that is subsequently combined with the aerosol propellant in a spray can or similar container. Suitable secondary fluid media may comprise organic solvents such as alcohols, glycols, ethers, or the like. Any of the organic solvents described herein as suitable for use in mechanically pumped or forced pressurization spray formulations may be incorporated in spray formulations containing an aerosol propellant as a secondary fluid medium as well.

Spray formulations comprising an organic solvent may comprise a mixture of organic solvents that evaporates in a specified period of time, typically under ambient conditions. In non-limiting examples, evaporation may take place in about 1 minute or less, or about 2 minutes or less, or about 5 minutes or less, or about 10 minutes or less, or about 15 minutes or less, or about 30 minutes or less. To facilitate evaporation, the metal nanoparticle agglomerates may be dispersed as a concentrate in a higher boiling organic solvent, such as a C₁₀ alcohol, which is then combined with a much larger quantity of low boiling organic solvent, such as ethanol or diethyl ether, optionally in further combination with additional organic solvents. The high boiling organic solvent may be sufficiently hydrophobic to facilitate dispersion of the metal nanoparticle agglomerates in the less hydrophobic and lower boiling organic solvent comprising the majority of the organic phase.

Spray formulations suitable for dispensation using a pump may be prepared by dispersing as-produced or as-isolated metal nanoparticles in an organic medium comprising one or more organic solvents. Optionally, one or more inorganic components may be present as well, particularly water. Particularly suitable organic solvents that may be used in a spray formulation include a C₁-C₁₁ alcohol, or multiple C₁-C₁₁ alcohols in any combination. C₁-C₄ alcohols may be particularly desirable due to their lower boiling points, which may facilitate solvent removal from a surface undergoing disinfection. Additional alcohol-miscible organic solvents may also be present. Ketone and aldehyde organic solvents in the C₂-C₁₁ size range may also be used, either alone or in combination with one or more alcohols. Ketone and aldehyde solvents are less polar than are alcohols and may aid in metal nanoparticle dispersion. Low boiling ethers such as diethyl ether, dipropyl ether, and diisopropyl ether, for example, may also be suitably used to promote metal nanoparticle dispersion. One or more glycol ethers (*e.g*., diethylene glycol, triethylene glycol, or the like), alkanolamines (*e.g.*, ethanolamine, triethanolamine, or the like), or any combination thereof may also be used alone or in combination with one or more alcohols or any of the other foregoing organic solvents. Various glymes may also be used similarly. Water-miscible organic solvents and mixtures of water and water-miscible organic solvents may be used as well, such as water-organic solvent mixtures comprising up to about 50% water by volume or up to about 75 % water by volume.

In various embodiments, individual metal nanoparticles within the metal nanoparticle agglomerates disposed upon a surface subject to biofilm formation may be about 20 nm or more in size, particularly wherein all or at least about 90 %, at least about 95 %, or at least about 99 % of the metal nanoparticles are about 20 nm to about 200 nm in size, or about 50 nm to about 250 nm in size, or about 20 nm to about 250 nm in size. Smaller copper nanoparticles (under 20 nm) may tend to undergo more extensive oxidation than do larger metal nanoparticles. By using larger metal nanoparticle agglomerates, the rate of oxidation may be more easily regulated upon a surface subject to biofilm formation and proliferation. For example, smaller copper nanoparticles may tend to undergo more extensive oxidation into CuO or Cu₂O than do larger copper nanoparticles having a size above 20 nm. Copper nanoparticles in a larger size range (20 nm or above) may afford a coating comprising a mixture of CuO and Cu₂O upon a metallic copper metal core, the combination of which may be advantageous for inactivating bacteria and preventing or limiting biofilm formation according to the disclosure herein. Silver nanoparticles in a similar size range may form a coating comprising silver oxide upon a metallic silver core. When copper nanoparticles and/or silver nanoparticles are agglomerated together upon a surface and are adhered thereto, the oxide coating may extend over at least a portion of the surface of the individual agglomerates, leaving an exposed copper or silver metal surface below within the porosity of the agglomerate. By having larger metal nanoparticles in the foregoing size range, a substantial amount of zero-valent metal may be retained for promoting biocidal activity, whereas smaller metal nanoparticles may form too much oxide to promote optimal bioactivity.

The foregoing notwithstanding, metal nanoparticles, such as copper nanoparticles, that are about 20 nm or less in size can also be used in the disclosure herein. Copper nanoparticles in this size range have a fusion temperature of about 220°C or below (*e.g*., a fusion temperature in the range of about 140°C to about 220°C) or about 200°C or below, or even about 175°C or below, which can provide advantages noted above. Silver nanoparticles about 20 nm or less in size may also be used in the disclosure herein and similarly exhibit a fusion temperature differing significantly from that of the corresponding bulk metal. Larger metal nanoparticles (either copper or silver nanoparticles), in turn, have a higher fusion temperature, which may rapidly increase and approach that of bulk metal as the nanoparticle size continues to increase. Even these larger metal nanoparticles may become adhered to the surface of an article once their surfactant coating has been lost and/or as bonding takes place via an adhesive, as described above. Optionally, bonding of the larger metal nanoparticles to the surface may take place by way of smaller metal nanoparticles may that have undergone fusion while contacting the larger metal nanoparticles within the metal nanoparticle agglomerates. Depending on the processing temperature and the fusion temperature of the copper nanoparticles and/or silver nanoparticles based upon their size, the metal nanoparticles may or may not be fused upon a surface when processed according to the disclosure herein. Regardless of whether the metal nanoparticles become fused or not once deposited upon a surface, after the surfactant coating is removed, the copper nanoparticles and/or silver nanoparticles may experience robust adherence to the surface and become effective for limiting formation of a biofilm once shed from the metal nanoparticle agglomerates in an active species form.

In addition to copper nanoparticles, silver nanoparticles, or alternative nanostructures containing these metals, other additives may be introduced upon a surface to aid in limiting the formation of biofilms according to the disclosure herein. Suitable additives may include, but are not limited to, those capable of producing reactive oxygen species (ROS), which may cause lipid, protein, or DNA damage in microorganisms, eventually leading to cell membrane damage and cell death. These additives may complement or enhance the biocidal activity conveyed by metal nanoparticle agglomerates containing copper nanoparticles, silver nanoparticles, or alternative metal nanoparticles having biocidal activity, such as those comprising zinc. Conventional disinfectant compounds may be included in the spray formulations as well, provided that they are compatible with a given use indication of the articles. For example, disinfectants may not be suitable for use when forming a surface coating upon an article destined for implantation *in vivo.*

NiO may be included as an additive upon the surface of an article in addition to metal nanoparticles. NiO is very efficient in producing ROS when present in small concentrations. NiO may be effective when included at, for example, about 0.5% to about 10% of the load of copper nanoparticles and/or silver nanoparticles upon the surface of an article (*e.g.*, 0.5 mg to 100 mg NiO) as sub-micron particles separate and distinct from the copper nanoparticles and/or silver nanoparticles. At these loadings, NiO is very effective against certain bacteria, which may broaden the biocidal effectiveness of copper or silver. Bismuth, zinc, and tin oxides may be similarly effective at loadings of about 0.5% to about 10% of the mass of copper nanoparticles.

TiO₂ may be included as an additive upon the surface of an article in addition to metal nanoparticles. TiO₂ may catalyze the formation of hydroxyl radicals upon UV irradiation (*e.g*., in sunlight) when an article is located outdoors, for example. Atmospheric moisture or water in contact with the article may supply the source of water for producing the hydroxyl radicals by photooxidation. TiO₂ may be present at about 1% to about 25% of the load of copper nanoparticles and/or silver nanoparticles upon the surface of the article. The TiO₂ may likewise be present in the form of nanoparticles and/or micron-size particles (*e.g*., about 100 nm to about 5 microns).

Cobalt compounds may also be combined with metal nanoparticle agglomerates, either alone or in combination with any of the foregoing additives. More specifically, cobalt (II) salts like halide, carbonate, sulfate, oxide (CoO), and the like may be present in combination with metal nanoparticle agglomerates in the disclosure herein. Cobalt ions may bind hypoxia inducible factor-1 alpha to create hypoxic conditions. Cobalt ions may be effective for this purpose when included at, for example, about 0.1% to about 10% of the load of copper nanoparticles and/or silver nanoparticles as sub-micron particles separate and distinct from the copper nanoparticles and/or silver nanoparticles. Cobalt nanoparticles may be present as well.

Copper nanoparticles and/or silver nanoparticles, including oxidized forms thereof, and NiO and/or TiO₂ and/or ZnO may also be used in any combination with one another as well. These additives may be introduced to the surface of an article at the same time as metal nanoparticles are introduced or at different times.

After depositing a spray formulation or an alternative coating formulation upon the surface of an article subject to biofilm formation, removal of the solvent and optionally the surfactants may take place. Although solvents and surfactants may be removed under ambient conditions (room temperature and atmospheric pressure), application of at least one of heating, gas flow, and/or vacuum (reduced pressure) may accelerate removal of the solvent and surfactants from the surface, thereby leading to the metal nanoparticle agglomerates becoming adhered to the surface. Heating may take place at any temperature up to or beyond the fusion temperature of the metal nanoparticles, provided that the heating temperature is not so high that the article itself experiences thermal damage. Thus, the metal nanoparticles may be fused or unfused when adhered to the surface of the article. Moreover, the heating temperature need not necessarily exceed the normal boiling point or reduced pressure boiling point of the surfactants and solvents in order to promote their removal. Gentle heating well below the boiling point of the surfactant and solvent may be sufficient to promote their removal in many instances. In non-limiting embodiments, the heating may be conducted under flowing nitrogen, air or other inert gas or under vacuum to promote removal. For example, heating the surface at a temperature of about 35°C to about 65°C in flowing nitrogen or air may be sufficient to remove the solvent and surfactant, thereby leaving unfused metal nanoparticles distributed upon at least a portion of the surface of an article as a plurality of metal nanoparticle agglomerates. Additional heating may be conducted thereafter, if desired, to promote at least partial metal nanoparticle fusion. In either case, after the surfactants are removed from the metal nanoparticle surface, robust adherence to the surface may be realized. When heating under higher temperatures, use of an inert atmosphere, such as nitrogen, may be desirable to limit degradation of article or article surface and/or to control the amount of surface oxidation taking place upon the metal nanoparticles once the surfactant coating has been removed.

Once the surfactant coating has been removed from the metal nanoparticles, particularly copper nanoparticles and/or silver nanoparticles, the metal nanoparticles and/or agglomerates thereof may undergo at least partial oxidation. As indicated above, in the case of copper nanoparticles, the size of the copper nanoparticles and the agglomerates thereof may be selected such that at least some copper metal remains following oxidation, since a mixture of copper metal (metallic copper) and oxidized copper may be beneficial for promoting inhibition or killing of bacteria. Silver nanoparticles may similarly experience different amounts of surface oxidation depending upon the size of the silver nanoparticles and how they are processed. In non-limiting embodiments, following surfactant removal, copper nanoparticles may form a reaction product within a surface comprising about 25% to about 99% metallic copper by weight, about 0.5% to about 60% Cu₂O by weight, and about 0.1% to about 20% CuO by weight. In more particular embodiments, the amount of metallic copper may be about 45% to about 90% by weight, or about 50% to about 70% by weight, and the amount of Cu₂O may about 10% by weight or less, such as about 0.1% to about 10% by weight or less or about 5% to about 10% by weight or less, and the amount of CuO may be about 1% by weight or less, such as about 0.1% to about 1% by weight or about 0.5% to about 1% by weight. The Cu₂O and CuO may form at least a partial coating (shell) or full coating upon the metal nanoparticles or agglomerates thereof that is about 10 nm or greater in thickness, or 100 nm or greater in thickness, such as about 100 nm to about 3 microns thick in many instances.

Silver nanoparticles adhered to the surface of an article subject to biofilm formation may similarly comprise about 25% to about 99% metallic silver by weight and the balance being Ag₂O. The Ag₂O may similarly be present in a coating (shell) having a thickness of about 10 nm or greater, such as about 100 nm to about 3 microns thick.

Accordingly, methods for inhibiting biofilm formation may comprise: providing an article having a surface with a coating thereon, the coating comprising metal nanoparticle agglomerates adhered to at least a portion of the surface; and exposing the article to conditions subject to promoting biofilm formation upon the surface. The coating at least partially inhibits formation or proliferation of a biofilm upon the surface of the article. The surface may contact a liquid medium at least periodically, wherein the liquid medium may encourage biofilm formation (*e.g.*, by forming a conditioning film upon the surface of the article). The methods may further comprise providing an uncoated article; applying metal nanoparticle agglomerates to the surface of the uncoated article; and adhering the metal nanoparticle agglomerates to the surface to form the coating. Adhering may comprise removing a surfactant coating from metal nanoparticles within the metal nanoparticle agglomerates, contacting metal nanoparticle agglomerates with an adhesive on the surface, or any combination thereof. Example techniques for at least partially removing a surfactant from the surface of metal nanoparticles may comprise, for instance, applying heat, gas flow, vacuum, or any combination thereof to the article after applying the metal nanoparticle agglomerates thereto.

Articles upon which metal nanoparticle agglomerates may be deposited as an at least partial coating are not believed to be particularly limited. Example articles may include those that are configured for *in vivo* implantation or skin contact (*e.g.*, medical devices or implants) or those configured for at least partial submersion or periodic contact with water during their operational use.

Illustrative examples of articles configured for *in vivo* implantation or skin contact and that may be protected against biofilm formation according to the disclosure herein may include medical devices, implants, prosthetics, bandages, and the like. More specific examples may include, for instance, temporary medical implants such as catheters, endotracheal tubes, nasogastric tubes, shunts, contact lenses, intrauterine devices, and the like; permanent or semi-permanent medical implants such as prosthetic joints, heart valves, stents, pacemakers, tympanostomy tubes, and the like; IV needles; tubing for dialysis machines or ventilators; skin grafting materials; and dental materials such as adhesives/amalgams, dentures, braces, retainers, and the like. In addition, targeted introduction of metal nanoparticle agglomerates to a subject having or potentially experiencing *in vivo* or external biofilm formation may also afford advantages. Examples may include patients having conditions such as ulcerative colitis, lung infections, irritable bowel disease, Crohn's disease, glycocalyx of the intestines, colorectal cancer due to microbial dysbiosis, gall stones, laryngitis, urinary tract infections, cystic fibrosis; external wounds, burns or ulcers; bed sores, or the like. While inhibiting or preventing biofilm formation, metal nanoparticle agglomerates may be applied directly to a site of biofilm formation (*e.g.*, upon a patient's tissue), or they may be applied upon a bandage or similar article configured to contact a site of potential biofilm formation. Examples may include wound or burn bandages, compression stockings, debriding treatments or curative implants, and the like. In addition, biofilm formation in a medical setting upon surfaces such as IV poles, wheelchairs, bed railings, surgical instruments, and the like may be mitigated by applying a metal nanoparticle coating thereto according to the disclosure herein.

Additional articles in need of protection against biofilm formation may include those in industrial, residential or commercial settings that are at least partially submersible or experience periodic contact with water. Illustrative examples may include, but are not limited to, pipes; pipelines; wellbore equipment and wellbore liners; heat exchangers; duct work; papermaking equipment; industrial and potable water distribution lines and equipment; oil rigs and deep sea risers; ships; docks; piers; bridge or building piers; wastewater treatment plant components; gardening components; pools; hot tubs; spas; fountains; floors and decking; bath tubs; showers; toilets; aquariums; food processing and storage facilities and containers; kitchen and bathroom equipment such as sponges and brushes; children's toys; water heaters; showers and faucets; rain gutters; water dispensers for livestock; and the like. Wood treated with metal nanoparticle agglomerates may supplement or be used as alternative to conventional pressure-treated wood for exterior uses, such as fences, piers, and the like.

In addition, metal nanoparticle agglomerates may also be applied directly to foodstuffs to limit spoilage promoted by biofilm-forming bacteria. When applied to agricultural products, such as grapes and other fruits and vegetables that spoil easily, the metal nanoparticle agglomerates may or may not be present with an adhesive, depending the desired ease of removal of the metal nanoparticle agglomerates from the foodstuff. Application of the metal nanoparticles to the fruits and vegetables may be conducted before or after harvesting. In still another example, metal nanoparticle agglomerates may be used to treat red tides and similar bacterial outbreaks.

Examples of biofilm-forming bacteria whose growth may be inhibited through the disclosure herein include many common types of bacteria, including both gram-positive and gram-negative varieties. This behavior is very unusual, as many antimicrobial agents are only active against one type of bacteria but not the other. Illustrative bacteria that may be inhibited from forming biofilms according to the disclosure herein include, for example, *Enterococcus faecalis, Staphylococcus aureus, Staphylococcus epidermidis, Streptococcus viridans, E. coli, Klebsiella pneumoniae, Proteus mirabilis and Pseudomonas aeruginosa.*

To facilitate a better understanding of the present disclosure, the following examples of preferred or representative embodiments are given.

### EXAMPLES

**Nitrile Glove Coating:** An acetone/isopropanol suspension containing 3% w/w copper nanoparticles is applied to the wet nitrile glove layer when emerging from a dip coating bath. Application is made using a commercially available spray setup with an application tip that is 1.8 mm or less in diameter. Going back and forth, the material is sprayed at a distance of 10.16 cm to 15.24 cm (4-6") away from the surface on the glove's palm side in single steady strokes with about a 10 % overlap, one pass only. After polymerization, the copper nanoparticles are embedded in the surface of the glove with a well dispersed load of about 3 mg/in².

**Thin Copper Layer Coating on Metal:** An acetone/isopropanol suspension containing 6% w/w copper nanoparticles and 0.4% w/w of an epoxy binder is applied to a solid surface using a commercially available airless sprayer (e.g., Graco Ultra 2000) using an application tip that is 1.8 mm or less in diameter. Going back and forth, the material is sprayed at a distance of 10.16 cm to 15.24 cm (4-6") away from the surface in single steady strokes with about a 10 % overlap, one pass only. At a layer thickness of 50-100 nm, 29.6 mL (1 fluid ounce) of such an ink can cover approximately 11.6 to 23.2 m² (125-250 ft²) of surface area. The resultant coating has a well dispersed copper load of about 0.78 to 0.93 mg/cm² (about 5-6 mg/in²).

**Thick Copper Layer Coating on Metal:** A paint containing 84% w/w copper nanoparticles in an alcohol binder mixture is applied to a solid surface using a sponge or paintbrush in form of a thick layer. After drying 24 h in air, a tack free surface is ready to be touched. At a layer thickness of 500 microns, 29.6 mL (1 fluid ounce) of such a paint can cover approximately 2.3 m² (25 ft²) of surface area. The resultant solid copper coating covers the entire surface.

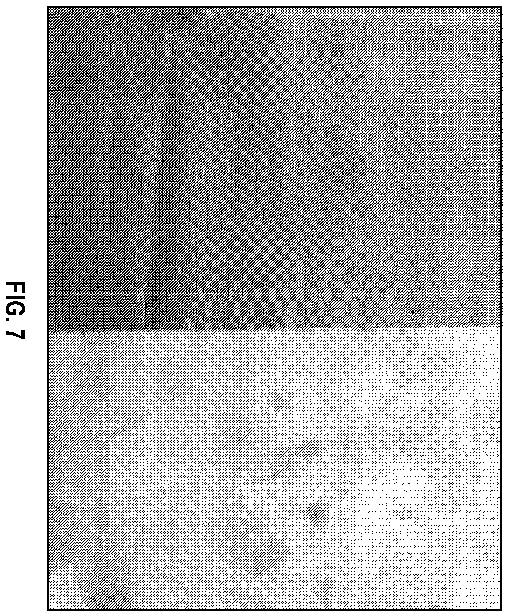

**Fabric Coating #1:** Agglomerates of copper nanoparticles in the 50-250 nm size range with a monolayer of amine surfactants on their surfaces and having an agglomerate size of 1-35 microns were adhered to a 55/45 cellulose/polyester fabric blend with an average fiber diameter of about 10 microns using an epoxy adhesive. This may be done via spray coating a suitable ink or dye formulation onto the fibers, or dip coating or gravure coating via a commercial process. The adhesive layer was about 20-50 nm thick, and the metal nanoparticle agglomerates were partially embedded in the adhesive layer with a substantial portion still exposed. The areal coverage of the agglomerates upon the fiber surfaces was about 20-50 %. The copper loading upon the fabric ranged from about 0.19 mg/cm² (about 1.2 mg/in²) to about 0.42 mg/cm² (about 2.7 mg/in²). Depending on size, some of the agglomerates may have the surfactant layer partially removed, thereby resulting in partial oxidation and an overall mixture of copper metal, Cu₂O and CuO species on the fiber surface. The copper metal to oxide ratio may reside in the 1-10 % range. Over time, oxidation and dissolution progressively result in fading of the initial dark brown-red color to more light yellow-green. FIG. 7 shows an illustrative photographic image of a fabric having agglomerates of copper nanoparticles adhered thereto, as fabricated (left side of image) and after extended use (right side of image). The nanoparticle-loaded fabric was then subjected to various stability and toxicological tests specified below.

**Fabric Coating #2:** Agglomerates of copper nanoparticles in the 20-150 nm size range with a partially removed monolayer of amine surfactants on their surfaces and having an agglomerate size of 5-15 microns were adhered to a 30/70 cellulose/polyester fabric blend with an average fiber diameter of about 10 microns using an epoxy adhesive. This may be done via spray coating a suitable ink or dye formulation onto the fibers, or dip coating or gravure coating via a commercial process. The adhesive layer was about 50-100 nm thick and the metal nanoparticle agglomerates were partially embedded in the adhesive layer with a substantial portion still exposed. The areal coverage of the agglomerates upon the fiber surfaces was about 30-70%. The copper loading upon the fabric ranged from about 0.357 cm/cm² (about 2.3 mg/in²) to about 0.7 mg/cm² (about 4.5 mg/in²). Depending on size, some of the agglomerates may be fully or partially oxidized, thereby resulting in an overall mixture of copper metal, Cu₂O and CuO species on the fiber surface. The copper metal to oxide ratio may reside in the 5-25 % range.

**Fabric Coating #3:** Agglomerates of copper nanoparticles in the 50-250 nm size range with a monolayer of amine surfactants on their surfaces and having an agglomerate size of 1-35 microns were adhered to a 55/45 cellulose/polyester fabric blend with an average fiber diameter of about 10 microns using a styrene acrylic acid block copolymer adhesive. This may be done via spray coating a suitable ink or dye formulation onto the fibers, or dip coating or gravure coating via a commercial process. The adhesive layer was about 100-250 nm thick, and the metal nanoparticle agglomerates were partially embedded in the adhesive layer with a substantial portion still exposed. The areal coverage of the agglomerates upon the fiber surfaces was about 10-35 %. The copper loading upon the fabric ranged from about 0.26 mg/cm² (about 1.7 mg/in²) to about 0.54 mg/cm² (about 3.5 mg/in²). Depending on size, some of the agglomerates may be fully or partially oxidized, thereby resulting in an overall mixture of copper metal, Cu₂O and CuO species on the fiber fabric surface. The copper metal to oxide ratio may reside in the 5-15 % range.

**Fabric Coating #4:** Agglomerates of copper nanoparticles in the 50-200 nm size range with a monolayer of amine surfactants on their surfaces and having an agglomerate size of 1-35 microns were adhered to a 100 % polypropylene fabric (melt-blown) with an average fiber diameter of about 10 microns using an epoxy adhesive. This may be done via spray coating a suitable ink or dye formulation onto the fibers, or dip coating or gravure coating via a commercial process. The adhesive layer was about 35-150 nm thick, and the metal nanoparticle agglomerates were partially embedded in the adhesive layer with a substantial portion still exposed. The areal coverage of the agglomerates on the fiber surfaces was about 5-30 %. The copper loading upon the fabric ranged from about 0.11 mg/cm² (about 0.7 mg/in²) to 0.25 mg/cm² (about 1.6 mg/in²). Depending on size, some of the agglomerates may be fully or partially oxidized, thereby resulting in an overall mixture of copper metal, Cu₂O and CuO species on the fiber surface. The copper metal to oxide ratio may reside in the 1-5 % range.

**Fabric Coating #5:** Agglomerates of copper nanoparticles in the 35-200 nm size range with a monolayer of amine surfactants on their surfaces and having an agglomerate size of 3-25 microns were adhered to a 100 % cotton fabric with an average fiber diameter of about 10 microns using a styrene acrylic acid block copolymer adhesive. This may be done via spray coating a suitable ink or dye formulation onto the fibers, or dip coating or gravure coating via a commercial process. The adhesive layer was about 50-150 nm thick, and the metal nanoparticle agglomerates were partially embedded in the adhesive layer with a substantial portion still exposed. The areal coverage of the agglomerates upon the fiber surfaces was about 40-75 %. The copper loading upon the fabric ranged from about 0.42 mg/cm² (about 2.7 mg/in²) to about 0.7 mg/cm² (about 4.5 mg/in²). Depending on size, some of the agglomerates may be fully or partially oxidized, thereby resulting in an overall mixture of copper metal, Cu₂O and CuO species on the fiber surface. The copper metal to oxide ratio may be in the 3-25 % range.

When the foregoing fabrics were utilized as dry wipes for disinfection of a hard surface, wiping the hard surface for just 5 seconds may result in full sterilization of a wide range of microbes, viruses and bacteria. Depending on the frequency of use, such dry wipes may remain effective for up to about 30 days. After use, the dry wipes may self-sterilize (e.g., in about 5 minutes or less) for frequent and rapid reuse. Since the pathogens are killed or inactivated upon contact, transfer and cross-contamination is unlikely to occur. The fabric is protected from biofilm formation, even when remaining wet (e.g., damp kitchen towels or bath towels). Further, no bacterial transfer from surface to surface occurs.

**Stability testing.** A 15.24 cm x 15.24 cm (6" x 6") sheet of fabric was tumbled in water for 8 hours. Only 1.4 % of the available copper by weight (0.54 mg) was released into the water.

Shedding was also determined by exposing the fabric to simulated breathing conditions (8.4 and 40.8 m/min face velocity gas flow) and analyzing a filter trap for liberated copper by SEM or EDS. The shedding tests did not reveal detectable liberation of copper from the fabric.

**VOCs.** No volatile organic compounds (VOCs) from a battery of 70 standard VOCs were detected as being released from the fabric when tested under standard conditions.

**Direct exposure to cell growth media.** A piece of fabric was first soaked in supplemented cell growth media for up to an hour and then removed. Thereafter, Vero cells or Calu-3 lung epithelial cells were immersed in the cell growth media and incubated overnight in a CO₂ incubator. Cell viability was determined by assessing ATP production using a luminescence assy. The luminescence assay did not reveal a substantial change in cell viability.

**Efficacy.** Efficacy of the fabric against a panel of bacterial and viral pathogens was tested. The panel included gram-positive, gram-negative, and antibiotic-resistant bacteria, bacteriophages as representatives of non-enveloped viruses, enveloped viruses such as H1N1 flu, H3N2 flu, and SARS-CoV-2, and non-enveloped viruses such as feline calicivirus. In all cases, >99 % kill rates were observed within 30 seconds, and full efficacy was maintained over at least 15 days of repeated daily exposure. The efficacy was >99.9 % over a standard EPA exposure time of 2 hours against *Staphylococcus aureus* (ATCC 6538), *Enterobacter aerogenes* (ATCC 13048), *Pseudomonas aeruginosa* (ATCC 15442), Methicillin Resistant *Staphylococcus aureus* MRSA (ATCC 33592), and *Escherichia coli* O157:H7 (ATCC 35150). The fabric maintained substantially 100% of the original efficacy against repeated viral inocculations (27M PFUs; H1N1, H3N2 and feline calicivirus) or bacterial loads introduced to the fabric over the course of 30 days. The fabric maintained >99.9 % efficacy against *Staphylococcus aureus* and *Klebsiella aerogenes* after months of daily high-touch use and moisture exposure with visible wear. An inactivation rate of substantially 100 % was realized against human wound pathogens such as *Acinetobacter baumannii, Klebsiella pneumonia, Pseudomonas aeruginosa, Enterococcus faecalis,* Methicillin-resistant *Staphylococcus aureus* (MRSA), and *Staphylococcus epidermidis* over 24 hours.

Unless otherwise indicated, all numbers expressing quantities of ingredients, properties such as molecular weight, reaction conditions, and so forth used in the present specification and associated claims are to be understood as being modified in all instances by the term "about." At the very least, each numerical parameter should at least be construed in light of the number of reported significant digits and by applying ordinary rounding techniques.

One or more illustrative embodiments incorporating the features of the present disclosure are presented herein. Not all features of a physical implementation are described or shown in this application for the sake of clarity. It is understood that in the development of a physical embodiment incorporating the present disclosure, numerous implementation-specific decisions must be made to achieve the developer's goals, such as compliance with system-related, business-related, government-related and other constraints, which vary by implementation and from time to time. While a developer's efforts might be time-consuming, such efforts would be, nevertheless, a routine undertaking for those of ordinary skill in the art and having benefit of this disclosure.

Therefore, the present disclosure is well adapted to attain the ends and advantages mentioned as well as those that are inherent therein. The particular embodiments disclosed above are illustrative only. It is therefore evident that the particular illustrative embodiments disclosed above may be altered within the scope of the present invention as defined in the claims. The disclosure herein suitably may be practiced in the absence of any element that is not specifically disclosed herein and/or any optional element disclosed herein. While compositions and methods are described in terms of "comprising," "containing," or "including" various components or steps, the compositions and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the element that it introduces.

## Claims

1. A biofilm-resistant article, comprising: a surface subject to biofilm formation that is adapted to contact a liquid medium at least periodically; and a coating comprising metal nanoparticle agglomerates adhered to at least a portion of the surface;
wherein the metal nanoparticle agglomerates comprise a plurality of metal nanoparticles having a surfactant coating thereon;
wherein the metal nanoparticles are fused, partially fused, and/or unfused and are associated with one another upon the surface; and
wherein the metal nanoparticles comprise copper nanoparticles and Cu₂O formed from at least partial oxidation of the copper nanoparticles.

2. The biofilm-resistant article of claim 1, wherein the surface is present upon a medical device or implant configured for implantation *in vivo* or application to an external tissue of a patient; or wherein the surface is present upon a structure that is at least partially submersible or experiences periodic contact with water.

3. The biofilm-resistant article of claim 1, wherein:
a) the metal nanoparticle agglomerates further comprise NiO, ZnO, TiO₂, a cobalt salt, or any combination thereof; and/or
b) the coating further comprises at least one metal salt, optionally wherein the at least one metal salt compound comprises a surfactant-stabilized metal salt compound.

4. The biofilm-resistant article of claim 1, wherein at least a portion of the metal nanoparticles are unfused with one another when the metal nanoparticle agglomerates are adhered to the surface, or wherein at least a majority of the metal nanoparticles range from 50 nm to 250 nm in size.

5. The biofilm-resistant article of claim 1, wherein:
a) the copper nanoparticles comprise 25 % to 99 % metallic copper by weight, 0.5 % to 60 % Cu₂O by weight, and 0.1 % to 20 % CuO by weight; or
b) the copper nanoparticles comprise 45 % to 90 % metallic copper by weight, 0.5 % to 60 % Cu₂O by weight, and 0.1 % to 20 % CuO by weight.

6. The biofilm-resistant article of claim 1, wherein the metal nanoparticle agglomerates range from 1 micron to 35 microns in size; or wherein the metal nanoparticle agglomerates are present at a coverage density of 0.16 mg / cm² to 0.76 mg / cm² (1 mg/in² to 5 mg/in²) upon the surface.

7. The biofilm-resistant article of claim 1, wherein the coating further comprises an adhesive, optionally wherein the adhesive is biologically compatible.

8. The biofilm-resistant article of claim 1, wherein the article is adapted to contact a flowing liquid medium.

9. The biofilm resistant article of claim 1, wherein the surface is selected from glass, metal, ceramic, polymers, cement, and textiles; or wherein the surface is present upon a structure that is at least partially submersible or experiences periodic contact with water, wherein the structure is selected from pipes, pipelines, wellbore equipment and wellbore liners, heat exchangers, duct work, industrial and potable water distribution lines and equipment, oil rigs and deep sea risers, and wastewater treatment plant components.

10. The biofilm resistant article of claim 1, wherein the coating comprising metal nanoparticle agglomerates provides activity against biofilm formation for at least 7 days, preferably wherein the coating comprising metal nanoparticle agglomerates provides activity against biofilm formation for at least 14 days, and more preferably wherein the coating comprising metal nanoparticle agglomerates provides activity against biofilm formation for at least 30 days.

11. A method comprising: providing a biofilm resistant article according to any of claims 1 to 8; and exposing the article to conditions subject to promoting biofilm formation upon the surface; wherein the coating at least partially inhibits formation or proliferation of a biofilm upon the surface of the article.

12. The method of claim 11, further comprising: providing an uncoated article; applying the metal nanoparticle agglomerates to at least a portion of the surface of the uncoated article; and adhering the metal nanoparticle agglomerates to the surface to form the coating.

13. The method of claim 12, wherein adhering comprises removing a surfactant coating from metal nanoparticles within the metal nanoparticle agglomerates, contacting the metal nanoparticle agglomerates with an adhesive on the surface, or any combination thereof, preferably wherein removing the surfactant coating comprises applying heat, gas flow, vacuum, or any combination thereof to the article after applying the plurality of metal nanoparticle agglomerates thereto.

14. The method of claim 11, wherein the conditions subject to promoting biofilm formation upon the surface comprise at least periodic contact with a liquid medium, optionally wherein the liquid medium is flowing.

15. The method of claim 11, wherein the surface is present upon a structure that is at least partially submersible or experiences periodic contact with water, wherein the structure is selected from pipes, pipelines, wellbore equipment and wellbore liners, heat exchangers, duct work, industrial and potable water distribution lines and equipment, oil rigs and deep sea risers, and wastewater treatment plant components.

## Patentansprüche

1. Biofilmresistenter Gegenstand, umfassend: eine Oberfläche, die einer Biofilmausbildung unterliegt, die dazu ausgelegt ist, mindestens periodisch mit einem flüssigen Medium in Kontakt zu kommen; und eine Beschichtung, die Metallnanopartikel-Agglomerate umfasst, die an mindestens einem Teil der Oberfläche haften;
wobei die Metallnanopartikel-Agglomerate eine Vielzahl von Metallnanopartikeln umfassen, die eine Tensidbeschichtung darauf aufweisen;
wobei die Metallnanopartikel verschmolzen, teilweise verschmolzen und/oder unverschmolzen sind und miteinander auf der Oberfläche verbunden sind; und
wobei die Metallnanopartikel Kupfernanopartikel und Cu₂O umfassen, das mindestens teilweise aus Oxidation der Kupfernanopartikel ausgebildet ist.

2. Biofilmresistenter Gegenstand nach Anspruch 1, wobei die Oberfläche auf einer medizinischen Vorrichtung oder einem Implantat vorhanden ist, die bzw. das zur Implantation *in vivo* oder zur Anwendung auf einem äußeren Gewebe eines Patienten konfiguriert ist; oder wobei die Oberfläche auf einer Struktur vorhanden ist, die mindestens teilweise eintauchbar ist oder periodischen Kontakt mit Wasser erfährt.

3. Biofilmresistenter Gegenstand nach Anspruch 1, wobei:
a) die Metallnanopartikel-Agglomerate ferner NiO, ZnO, TiO₂, ein Kobaltsalz oder irgendeine Kombination davon umfassen; und/oder
b) die Beschichtung ferner mindestens ein Metallsalz umfasst, wobei optional die mindestens eine Metallsalzverbindung eine tensidstabilisierte Metallsalzverbindung umfasst.

4. Biofilmresistenter Gegenstand nach Anspruch 1, wobei mindestens ein Teil der Metallnanopartikel miteinander unverschmolzen ist, wenn die Metallnanopartikel-Agglomerate an die Oberfläche angehaftet sind, oder wobei mindestens eine Mehrzahl der Metallnanopartikel in einem Größenbereich von 50 nm bis 250 nm liegen.

5. Biofilmresistenter Gegenstand nach Anspruch 1, wobei:
a) die Kupfernanopartikel 25 Gew.-% bis 99 Gew.-% metallisches Kupfer, 0,5 Gew.-% bis 60 Gew.-% Cu₂O und 0,1 Gew.-% bis 20 Gew.-% CuO umfassen; oder
b) die Kupfernanopartikel 45 Gew.-% bis 90 Gew.-% metallisches Kupfer, 0,5 Gew.-% bis 60 Gew.-% Cu₂O und 0,1 Gew.-% bis 20 Gew.-% CuO umfassen.

6. Biofilmresistenter Gegenstand nach Anspruch 1, wobei die Metallnanopartikel-Agglomerate in einem Größenbereich von 1 Mikron bis 35 Mikron liegen; oder wobei die Metallnanopartikel-Agglomerate bei einer Bedeckungsdichte von 0,16 mg/cm² bis 0,76 mg/cm² (1 mg/in² bis 5 mg/in²) auf der Oberfläche vorhanden sind.

7. Biofilmresistenter Gegenstand nach Anspruch 1, wobei die Beschichtung ferner einen Klebstoff umfasst, wobei optional der Klebstoff biologisch verträglich ist.

8. Biofilmresistenter Gegenstand nach Anspruch 1, wobei der Gegenstand dazu ausgelegt ist, mit einem fließenden flüssigen Medium in Kontakt zu kommen.

9. Biofilmresistenter Gegenstand nach Anspruch 1, wobei die Oberfläche ausgewählt ist aus Glas, Metall, Keramik, Polymeren, Zement und Textilien; oder wobei die Oberfläche auf einer Struktur vorhanden ist, die mindestens teilweise eintauchbar ist oder periodischen Kontakt mit Wasser erfährt, wobei die Struktur ausgewählt ist aus Rohren, Rohrleitungen, Bohrungsausrüstung und Bohrungsauskleidungen, Wärmetauschern, Kanalsystemen, industriellen und Trinkwasserverteilungsleitungen und -ausrüstung, Ölplattformen und Tiefseesteigrohren, und Abwasserbehandlungsanlagenkomponenten.

10. Biofilmresistenter Gegenstand nach Anspruch 1, wobei die Beschichtung, die Metallnanopartikel-Agglomerate umfasst, eine Wirkung gegen Biofilmausbildung für mindestens 7 Tage bereitstellt, wobei vorzugsweise die Beschichtung, die Metallnanopartikel-Agglomerate umfasst, eine Wirkung gegen Biofilmausbildung für mindestens 14 Tage bereitstellt, und mehr bevorzugt, wobei die Beschichtung, die Metallnanopartikel-Agglomerate umfasst, eine Wirkung gegen Biofilmausbildung für mindestens 30 Tage bereitstellt.

11. Verfahren, umfassend: Bereitstellen eines biofilmresistenten Gegenstands nach einem der Ansprüche 1 bis 8; und Aussetzen des Artikels gegenüber Bedingungen, die dazu geeignet sind, die Biofilmausbildung auf der Oberfläche zu fördern; wobei die Beschichtung die Ausbildung oder Proliferation eines Biofilms auf der Oberfläche des Gegenstands mindestens teilweise hemmt.

12. Verfahren nach Anspruch 11, ferner umfassend: Bereitstellen eines unbeschichteten Gegenstands; Anwenden der Metallnanopartikel-Agglomerate auf mindestens einen Teil der Oberfläche des unbeschichteten Gegenstands; und Anhaften der Metallnanopartikel-Agglomerate an die Oberfläche, um die Beschichtung auszubilden.

13. Verfahren nach Anspruch 12, wobei das Anhaften das Entfernen einer Tensidbeschichtung von Metallnanopartikeln innerhalb der Metallnanopartikel-Agglomerate, das Inkontaktkommen der Metallnanopartikel-Agglomerate mit einem Klebstoff auf der Oberfläche oder irgendeine Kombination davon umfasst, wobei vorzugsweise das Entfernen der Tensidbeschichtung das Anwenden von Wärme, Gasströmung, Vakuum oder irgendeiner Kombination davon auf den Gegenstand nach dem Anwenden der Vielzahl von Metallnanopartikel-Agglomeraten darauf umfasst.

14. Verfahren nach Anspruch 11, wobei die Bedingungen, die dazu geeignet sind, die Biofilmausbildung auf der Oberfläche zu fördern, mindestens periodischen Kontakt mit einem flüssigen Medium umfassen, wobei optional das flüssige Medium strömend ist.

15. Verfahren nach Anspruch 11, wobei die Oberfläche auf einer Struktur vorhanden ist, die mindestens teilweise eintauchbar ist oder periodischen Kontakt mit Wasser erfährt, wobei die Struktur ausgewählt ist aus Rohren, Rohrleitungen, Bohrungsausrüstung und Bohrungsauskleidungen, Wärmetauschern, Kanalsystemen, industriellen und Trinkwasserverteilungsleitungen und -ausrüstung, Ölplattformen und Tiefseesteigrohren, und Abwasserbehandlungsanlagenkomponenten.

## Revendications

1. Article résistant à un biofilm, comprenant : une surface sujette à la formation de biofilm qui est conçue pour entrer en contact avec un milieu liquide au moins périodiquement ; et un revêtement comprenant des agglomérats de nanoparticules métalliques collés à au moins une partie de la surface ;
dans lequel les agglomérats de nanoparticules métalliques comprennent une pluralité de nanoparticules métalliques ayant un revêtement de tensioactif sur celles-ci ;
dans lequel les nanoparticules métalliques sont fusionnées, partiellement fusionnées et/ou non fusionnées et sont associées les unes aux autres sur la surface ; et
dans lequel les nanoparticules métalliques comprennent des nanoparticules de cuivre et du Cu₂O formé à partir d'une oxydation au moins partielle des nanoparticules de cuivre.

2. Article résistant à un biofilm selon la revendication 1, dans lequel la surface est présente sur un dispositif médical ou un implant conçu pour implantation *in vivo* ou application sur un tissu externe d'un patient ; ou dans lequel la surface est présente sur une structure qui est au moins partiellement submersible ou subit un contact périodique avec de l'eau.

3. Article résistant à un biofilm selon la revendication 1, dans lequel :
a) les agglomérats de nanoparticules métalliques comprennent en outre NiO, ZnO, TiO₂, un sel de cobalt, ou une combinaison quelconque de ceux-ci ; et/ou
b) le revêtement comprend en outre au moins un sel métallique, éventuellement dans lequel l'au moins un composé de sel métallique comprend un composé de sel métallique stabilisé par un tensioactif.

4. Article résistant à un biofilm selon la revendication 1, dans lequel au moins une partie des nanoparticules métalliques ne sont pas fusionnées les unes avec les autres lorsque les agglomérats de nanoparticules métalliques adhèrent à la surface, ou dans lequel au moins une majorité des nanoparticules métalliques ont une taille comprise entre 50 nm et 250 nm.

5. Article résistant à un biofilm selon la revendication 1, dans lequel :
a) les nanoparticules de cuivre comprennent 25 % à 99 % de cuivre métallique en poids, 0,5 % à 60 % de Cu₂O en poids, et 0,1 % à 20 % de CuO en poids ; ou
b) les nanoparticules de cuivre comprennent 45 % à 90 % de cuivre métallique en poids, 0,5 % à 60 % de Cu₂O en poids, et 0,1 % à 20 % de CuO en poids.

6. Article résistant à un biofilm selon la revendication 1, dans lequel les agglomérats de nanoparticules métalliques ont une taille comprise entre 1 micron et 35 microns ; ou dans lequel les agglomérats de nanoparticules métalliques sont présents à une densité de couverture de 0,16 mg/cm² à 0,76 mg/cm² (1 mg/in² à 5 mg/in²) sur la surface.

7. Article résistant à un biofilm selon la revendication 1, dans lequel le revêtement comprend en outre un adhésif, éventuellement dans lequel l'adhésif est biologiquement compatible.

8. Article résistant à un biofilm selon la revendication 1, dans lequel l'article est adapté pour entrer en contact avec un milieu liquide en écoulement.

9. Article résistant à un biofilm selon la revendication 1, dans lequel la surface est choisie parmi du verre, du métal, de la céramique, des polymères, du ciment, et des textiles ; ou dans lequel la surface est présente sur une structure qui est au moins partiellement submersible ou subit un contact périodique avec de l'eau, dans lequel la structure est choisie parmi des tuyaux, des pipelines, des équipements de puits de forage et des revêtements de puits de forage, des échangeurs de chaleur, des conduits, des lignes et des équipements de distribution d'eau industrielle et potable, des plates-formes pétrolières et des colonnes montantes en haute mer, et des composants d'usines de traitement des eaux usées.

10. Article résistant au biofilm selon la revendication 1, dans lequel le revêtement comprenant des agglomérats de nanoparticules métalliques fournit une activité contre la formation de biofilm pendant au moins 7 jours, de préférence dans lequel le revêtement comprenant des agglomérats de nanoparticules métalliques fournit une activité contre la formation de biofilm pendant au moins 14 jours, et plus préférablement dans lequel le revêtement comprenant des agglomérats de nanoparticules métalliques de nanoparticules fournit une activité contre la formation de biofilm pendant au moins 30 jours.

11. Procédé comprenant : la fourniture d'un article résistant à un biofilm selon l'une quelconque des revendications 1 à 8 ; et l'exposition de l'article à des conditions susceptibles de favoriser la formation d'un biofilm sur la surface ; dans lequel le revêtement inhibe au moins partiellement la formation ou la prolifération d'un biofilm sur la surface de l'article.

12. Procédé selon la revendication 11, comprenant en outre : la fourniture d'un article non revêtu ; l'application des agglomérats de nanoparticules métalliques sur au moins une partie de la surface de l'article non revêtu ; et la fixation par adhérence des agglomérats de nanoparticules métalliques à la surface pour former le revêtement.

13. Procédé selon la revendication 12, dans lequel l'adhésion comprend le retrait d'un revêtement tensioactif de nanoparticules métalliques à l'intérieur des agglomérats de nanoparticules métalliques, la mise en contact des agglomérats de nanoparticules métalliques avec un adhésif sur la surface, ou une combinaison quelconque de ceux-ci, de préférence dans lequel le retrait du revêtement tensioactif comprend l'application de chaleur, d'un flux de gaz, d'un vide ou de toute leur combinaison à l'article après l'application de la pluralité d'agglomérats de nanoparticules métalliques à celui-ci.

14. Procédé selon la revendication 11, dans lequel les conditions soumises à la promotion de la formation d'un biofilm sur la surface comprennent au moins un contact périodique avec un milieu liquide, éventuellement dans lequel le milieu liquide s'écoule.

15. Procédé selon la revendication 11, dans lequel la surface est présente sur une structure qui est au moins partiellement submersible ou subit un contact périodique avec de l'eau, dans lequel la structure est choisie parmi des tuyaux, des pipelines, des équipements de puits de forage et des revêtements de puits de forage, des échangeurs de chaleur, des conduits, des lignes et des équipements de distribution d'eau potable et industrielle, des plates-formes pétrolières et colonnes montantes en eaux profondes, et des composants d'usines de traitement des eaux usées.
